# FASCICULE DE BREVET EUROPEEN

(11) **EP 0 450 039 B1**
(45) Date de publication et mention de la délivrance du brevet: **05.06.1996**
(21) Numéro de dépôt: 90916034.3
(22) Date de dépôt: 17.10.1990
(51) Int. Cl.: C07D 241/44, C07D 401/06, A61K 31/495

(54) **DERIVES DE DIHYDRO-1,2 OXO-2 QUINOXALINES, LEUR PREPARATION ET LEUR APPLICATION EN THERAPEUTIQUE**
1,2-DIHYDRO-2-OXOQUINOXALINDERIVATE, DEREN HERSTELLUNG UND VERWENDUNG IN DER THERAPIE
DIHYDRO-1,2 OXO-2 QUINOXALINE DERIVATES, THEIR PREPARATION AND THEIR APPLICATION IN THERAPEUTICS

(30) Priorité: 23.10.1989 FR 8913961
(43) Date de publication de la demande: 09.10.1991
(73) Titulaire: PIERRE FABRE MEDICAMENT, F-92100 Boulogne (FR)
(72) Inventeur: PITET, Guy, F-31400 Toulouse (FR); FAURE, Christian, F-31200 Toulouse (FR); COURET, Françoise, F-31450 Corransac (FR); BIGG, Dennis, F-81100 Castres (FR); TARAYRE, Jean-Pierre, Valdurenque F-81100 Castres (FR)
(74) Mandataire: Doat, Jean Pierre
(86) Numéro de dépôt international: FR9000752
(87) Numéro de publication internationale: WO9105772

(56) Documents cités:
- EP-A- 0 032 564
- EP-A- 0 242 957
- US-A- 4 181 724

## Description

La présente invention a pour objet de nouveaux dérivés de dihydro-1,2 oxo-2 quinoxalines, leur préparation et leur application en thérapeutique.

US-A-4 181 724 décrit des dérivés de dihydro-1,2-oxo-2-quinoxaline qui possèdent des activités thérapeutiques telles que broncholytiques et antiasthmatiques.

Les composés de l'invention répondent à la formule générale I : 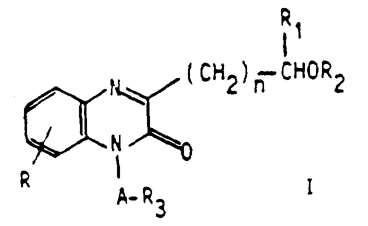 dans laquelle
- R: représente un atome d'hydrogène ou d'halogène
- R₁: représente un atome d'hydrogène ou un radical alkyle en C₁-C₄ droit ou ramifié
- R₂: représente :
- un atome d'hydrogène
- un radical C(O)R₄
   où R₄ est un groupe alkyle en C₁-C₄ droit ou ramifié
- un radical C(O)NHR₅
   où R₅ est un groupe alkyle en C₁-C₄ droit ou ramifié. ou un groupe phényle
- A: représente une chaîne alkyléne en C₁-C₄
- R₃: représente :
- un atome d'hydrogène, un radical alkyle en C₁-C₄, un radical cycloalkyle en C₃-C₆, un groupe alcynyle, nitrile, hydroxyle, carboxamido, pyridyle, phényle, ou phényle substitué par un atome d'halogène, un groupe alkyle en C₁-C₄, un groupe alkoxy en C₁-C₄, ou un groupe nitro
- un radical alcényle de formule : 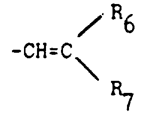 où R₆, R₇ peuvent être, indépendamment l'un de l'autre, un atome d'hydrogène, un groupe alkyle en C₁-C₄, un groupe phényle, ou un groupe alkoxycarbonyle
- un radical de formule :

   -OC(O)R₈

   où R₈ représente un radical alkyle en C₁-C₄
- un radical de formule :

   -OC(O)NHR₉

   où R₉ représente un groupe alkyle en C₁-C₄, droit ou ramifié, ou un groupe phényle
- n: peut prendre des valeurs de 2 à 4.

En outre, l'invention couvre les sels des composés de formule générale I avec des acides pharmaceutiquement acceptables dans le cas des composés présentant une basicité suffisante.

Les composés de formule générale I (R₂=H) de l'invention peuvent être préparés selon le schéma de réaction suivant :

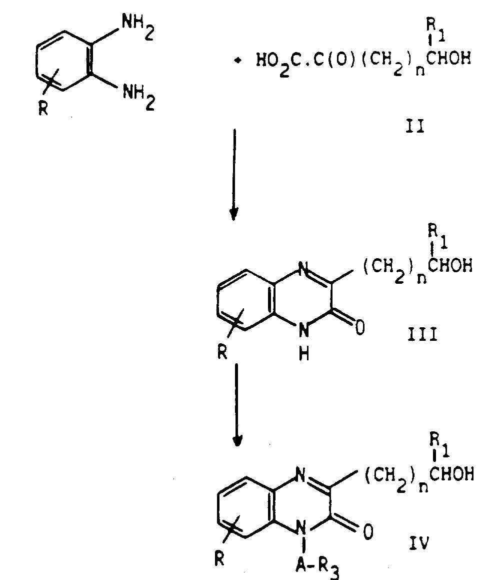

Les céto-acides de départ de formule II peuvent être obtenus selon des méthodes connues, par exemple, celle décrite par Korte et al., Chem.Ber. 92, 877-83 (1959).

La réaction du céto-acide de formule II avec une orthophénylène diamine s'effectue de préférence dans un solvant alcoolique tel que l'éthanol, ou un solvant acide, tel que l'acide acétique. La réaction peut être conduite à la température ambiante ou à une température allant jusqu'au point d'ébullition du solvant.

Les dihydro-1,2 oxo-2 quinoxalines de formule générale III ainsi obtenues sont ensuite traitées par un réactif R₃-A-X où R₃ et A sont définis comme dessus et où X représente un atome nucléofuge tel que l'iode; le chlore, le brome, ou un groupe mésylate ou tosylate, en présence d'une base tel que l'hydrure de sodium. La réaction est effectuée au sein d'un solvant aprotique tel que le DMF.

Les quinoxalines de formule IV ainsi obtenues correspondent aux composés de formule I où R₂ représente un atome d'hydrogène.

Pour préparer un composé de formule I où R₂ représente un groupe de formule -C(O)R₄ (R₄ étant défini comme ci-dessus), on fait réagir le composé de formule IV avec un excès d'anhydride de formule (R₄CO)₂O, de préférence à chaud. On peut également préparer un composé de formule I où R₂ représente un groupe de formule -C(O)R₄ en faisant réagir un composé de formule générale III avec un excès d'anhydride de formule (R₄CO)₂O et ensuite alkylant ce produit par un réactif de formule R₃-A-X d'une manière analogue à celle décrite ci-dessus.

Pour préparer un composé de formule I où R₂ représente un groupe de formule -C(O)NHR₅ (R₅ étant défini comme ci-dessus), on fait réagir un composé de formule IV avec un isocyanate de formule R₅NCO dans un solvant aprotique tel que le toluène à une température allant jusqu'au point d'ébullition du solvant, ou bien on fait réagir un composé de formule IV avec le phosgène dans un solvant aprotique, par exemple le toluène, et ensuite avec une amine de formule R₅NH₂.

Pour préparer un composé de formule I où R₃ représente un radical -OC(O)R₈ (R₈ étant défini comme ci-dessus), on peut faire réagir un composé de formule I où R₃ représente un groupe hydroxyle avec un excès d'anhydride de formule (R₈CO)₂O, de préférence à chaud.

Pour préparer un composé de formule I où R₃ représente un radical -OC(O)NHR₉ (R₉ étant défini comme ci-dessus), on peut faire réagir un composé de formule I où R₃ représente un groupe hydroxyle avec un isocyanate de formule R₉NCO au sein d'un solvant aprotique tel que le toluène ou le xylène à la température du reflux du solvant.

Les exemples suivants illustrent l'invention sans toutefois en limiter la portée.

Les analyses et les spectres RMN et IR confirment la structure des composés obtenus selon l'invention.

### Exemple 1

Phényl-1 (hydroxy-3 propyl)-3 dihydro-1,2 oxo-2 quinoxaline [I ; R = R₁ = R₂ = H, n = 2, A = CH₂, R₃ = CH=C(CH₃)₂] : composé 1.
**1.1** (hydroxy-3 propyl)-3 dihydro-1,2 oxo-2 quinoxaline
   A une solution de 43 g d'acide hydroxy-5 oxo-2 pentanoique dans 430 ml d'éthanol, on ajoute 35 g d'orthophénylène diamine. On agite une nuit à température ambiante, puis on évapore à sec sous vide. On dissout le résidu brun obtenu dans 400 ml de soude 2N. Après filtration sur un lit de célite, le filtrat est acidifié par 80 ml d'acide chlorhydrique concentré ; le solide brun clair est filtré, puis recristallisé dans 500 ml d'éthanol. Après une nuit au réfrigérateur, filtration puis séchage sous vide à 70°C, on isole 36,15 g de cristaux beiges de (hydroxy-3 propyl)-3 dihydro-1,2 oxo-2 quinoxaline.
   P.F. 200°C (Kofler)
   Rendement : 55 %
   IR (C=O) : 1650 cm⁻¹
**1.2** Prényl-1 (hydroxy-3 propyl)-3 dihydro-1,2 oxo-2 quinoxaline ; composé 1
   A une suspension de 9,68 g d'hydrure de sodium à 60 dans 50 ml de DMF anhydre, on ajoute lentement une solution de 44,9 g du (hydroxy-3 propyl)-3 dihydro-1,2 oxo-2 quinoxaline (obtenu selon 1.1) dans 450 ml de DMF. La solution est agitée jusqu'à la fin du dégagement d'hydrogène, puis on ajoute 36 g de bromure de prényle. On agite la solution obtenue une nuit à la température ambiante, puis on évapore à sec sous pression réduite. Le résidu brun est traité par 150 ml d'eau et 400 ml de chlorure de méthylène. Après évaporation sous vide, on obtient un solide beige qui est recristallisé dans 200 ml de toluène. On isole ainsi 33,8 g du composé 1 sous forme de cristaux jaune pâle.
   P.F. 97°C (Kofler)
   Rendement 56 %
   IR (C=O) : 1655 cm⁻¹

### Exemple 2

Allyl-1 (acétoxy-3 butyl)-3 dihydro-1,2 oxo-2 quinoxaline [I ; R = H, R₁ = CH₃, R₂ = C(O)CH₃, n = 2, A = CH₂, R₃ = -CH=CH₂] : composé 2.
**2.1** (acétoxy-3 butyl)-3 dihydro-1,2 oxo-2 quinoxaline
   Dans un ballon de 100 ml muni d'une agitation magnétique et d'un réfrigérant à reflux surmonté d'une garde à CaCl₂, on place 1 g de (hydroxy-3 butyl)-3 dihydro-1,2 oxo-2 quinoxaline obtenu par une méthode analogue à celle décrite pour l'exemple 1. On ajoute 10 ml d'anhydride acétique et on chauffe à reflux pendant deux heures. On laisse refroidir, et après l'addition de 10 ml d'eau, on chauffe à 60°C pendant 30 mn. Le solide obtenu après l'évaporation du solvant est recristallisé dans l'éthanol pour fournir 0,8 g du dérivé acétylé.
   P.F. 138°C (Kofler)
   IR (C=O) : 1665 cm⁻¹, 1680 cm⁻¹
**2.2** Allyl-1 (acétoxy-3 butyl)-3 dihydro-1,2 oxo-2 quinoxaline : composé 2
   On ajoute 0,75 g d'hydrure de sodium à 50 % à une solution de 3,9 g de (acétoxy-3 butyl)-3 dihydro-1,2 oxo-2 quinoxaline (obtenu selon 2.1) dans 100 ml de DMF. Après deux heures d'agitation, on ajoute 3 ml de bromure d'allyle et on maintient l'agitation pendant 16 heures. Le mélange réactionnel est concentré sous vide et dilué avec de l'eau avant d'extraire avec le chlorure de méthylène. L'huile brune obtenue après séchage et évaporation du chlorure de méthylène est purifiée par chromatographie sur gel de silice (élution : éther isopropylique puis éther éthylique) pour donner 3 g de composé 2 sous forme d'huile.
   Rendement : 66,7 %
   - IR :: 1660 cm⁻¹ (oxo-2)
   1735 cm⁻¹ (OC(O)CH₃)

### Exemple 3

Propyl-1 (méthylcarbamoyloxy-3 propyl)-3 dihydro-1,2 oxo-2 quinoxaline [I ; R = R₁ = H, R₂ = C(O)NHCH₃, n = 2, A = CH₂, R₃ = -CH₂CH₃] : composé 3
**Méthode A**
   On ajoute 16,2 g de propyl-1 (hydroxy-3 propyl)-3 dihydro-1,2 oxo-2 quinoxaline, obtenu d'une manière analogue à celle décrite pour le composé 1, à 80 ml d'une solution à 20 % de phosgène dans le toluène. On agite une heure à la température ambiante, puis on évapore à sec sous vide et on ajoute 25 ml d'une solution de méthylamine à 33 % dans l'éthanol et 100 ml de benzène anhydre. On agite le mélange réactionnel pendant une nuit à température ambiante, on évapore à sec et on reprend le résidu avec 100 ml d'eau et 200 ml de chlorure de méthylène.
   Après décantation, on sèche la phase organique sur sulfate de sodium, filtre, évapore à sec et recristallise le solide obtenu dans 100 ml d'isopropanol. On obtient 9,86 g de composé 3 sous forme de cristaux blancs.
   P.F. 115°C (Kofler)
   Rendement : 49,5 %
   - IR :: 1650 cm⁻¹ (oxo-2)
   1695 cm⁻¹ (carbamate)
**Méthode B**
   Dans un ballon de 250 ml équipé d'une agitation magnétique, d'un réfrigérant à reflux et d'une garde à CaCl₂, on place 5,7 g de propyl-1 (hydroxy-3 propyl)-3 dihydro-1,2 oxo-2 quinoxaline dissout dans 60 ml de toluène anhydre. On ajoute 1,58 g (1,64 ml) d'isocyanate de méthyle et on chauffe une nuit à reflux. On évapore à sec sous vide et on cristallise le solide obtenu dans l'éthanol On obtient 3 g de composé 3 sous forme de cristaux blancs. P.F. 115°C (Kofler)
   Rendement : 43 %
   - IR :: 1650 cm⁻¹ (oxo-2)
   1695 cm⁻¹ (carbamate)

### Exemple 4

(méthylcarbamoyloxy-2 éthyl)-1 (hydroxy-3 butyl)-3 dihydro-1,2 oxo-2 quinoxaline [I ; R = H, R₁ = CH₃, R₂ = H, n = 2, A = CH₂CH₂, R₃ = -OC(O)NHCH₃] : composé 4.

Dans un ballon de 250 ml équipé d'une agitation magnétique, d'un réfrigérant à reflux muni d'une garde à CaCl₂, on introduit une solution de 5,09 g de (hydroxy-2 éthyl)-1 (hydroxy-3 butyl)-3 dihydro-1,2 oxo-2 quinoxaline dans 50 ml de xylène anhydre. On ajoute 1,35 g (1,4 ml) d'isocyanate de méthyle et on chauffe à reflux pendant 3 heures. On évapore le solvant et le résidu obtenu est chromatographie sur gel de silice (éluant CH₂Cl₂/HeOH = 95/5). On isole la fraction correspondant à une Rf de 0,42 (silice CHCl₃/HeOH = 95/5) qui est ensuite cristallisé dans 30 ml de toluène pour donner 1,54 g de composé 4 sous forme de cristaux jaune pâle.
P.F. 136°C (Kofler)
Rendement : 25 %
- IR :: 1645 cm⁻¹ (oxo-2)
1700 cm⁻¹ (carbamate)

Le tableau ci-après résume les principaux produits synthétisés qui illustrent l'invention sans toutefois en limiter la portée.

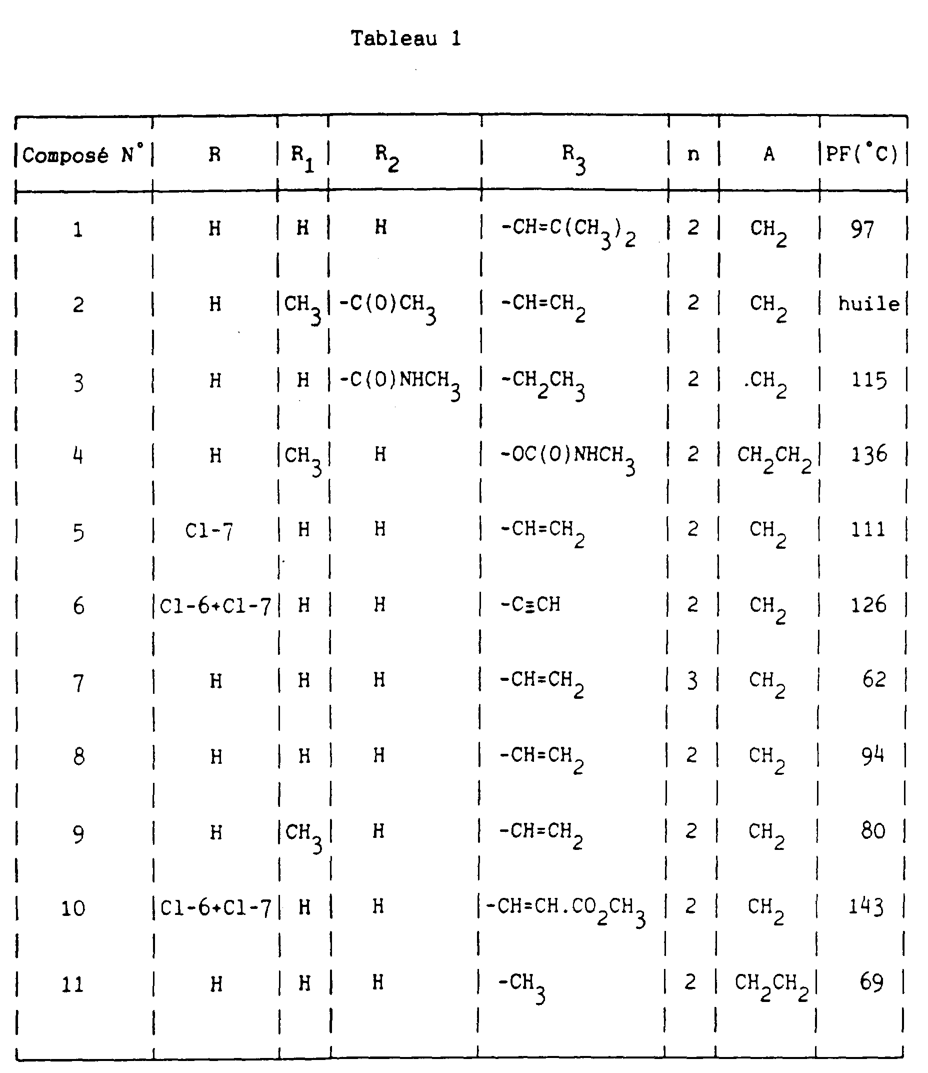

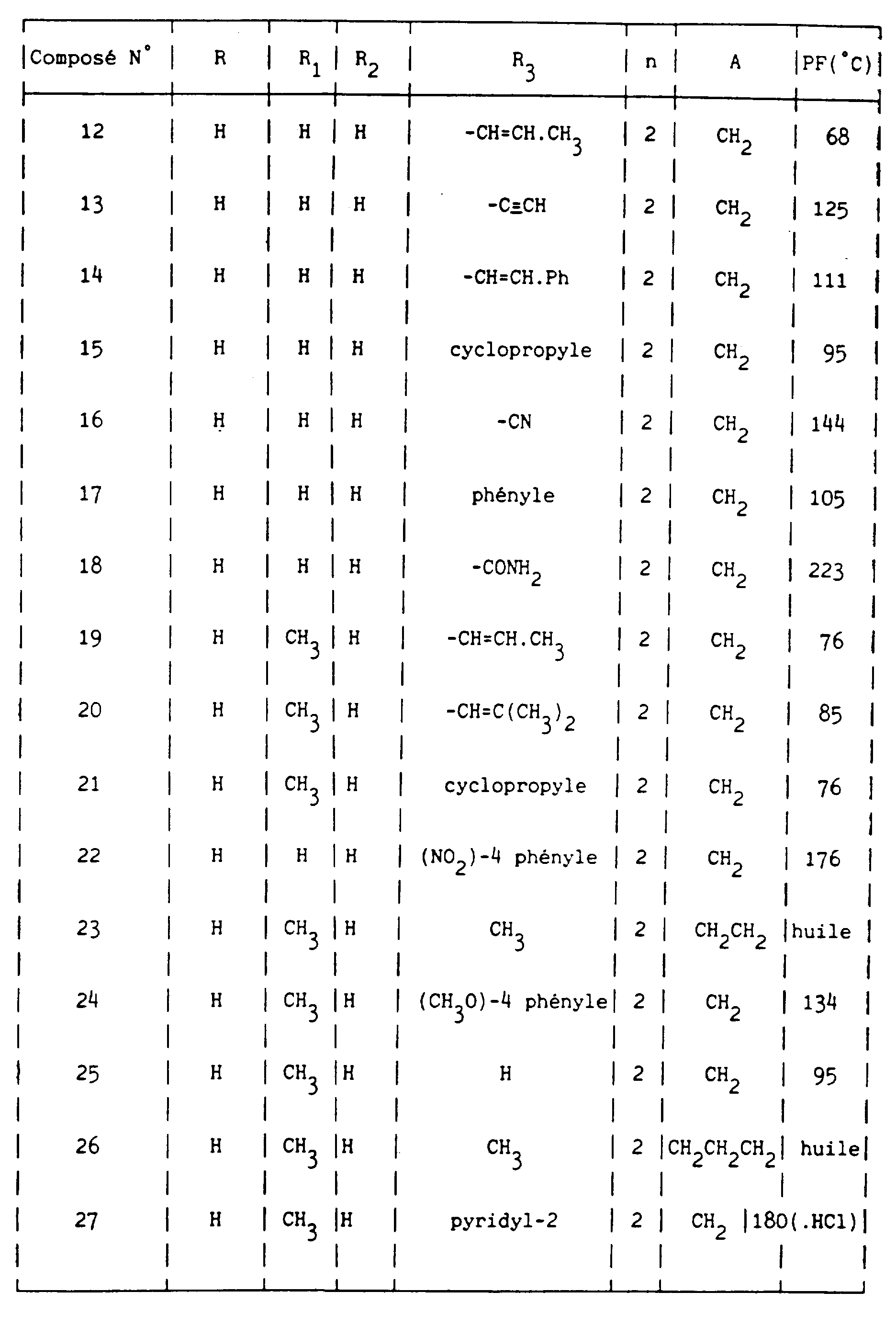

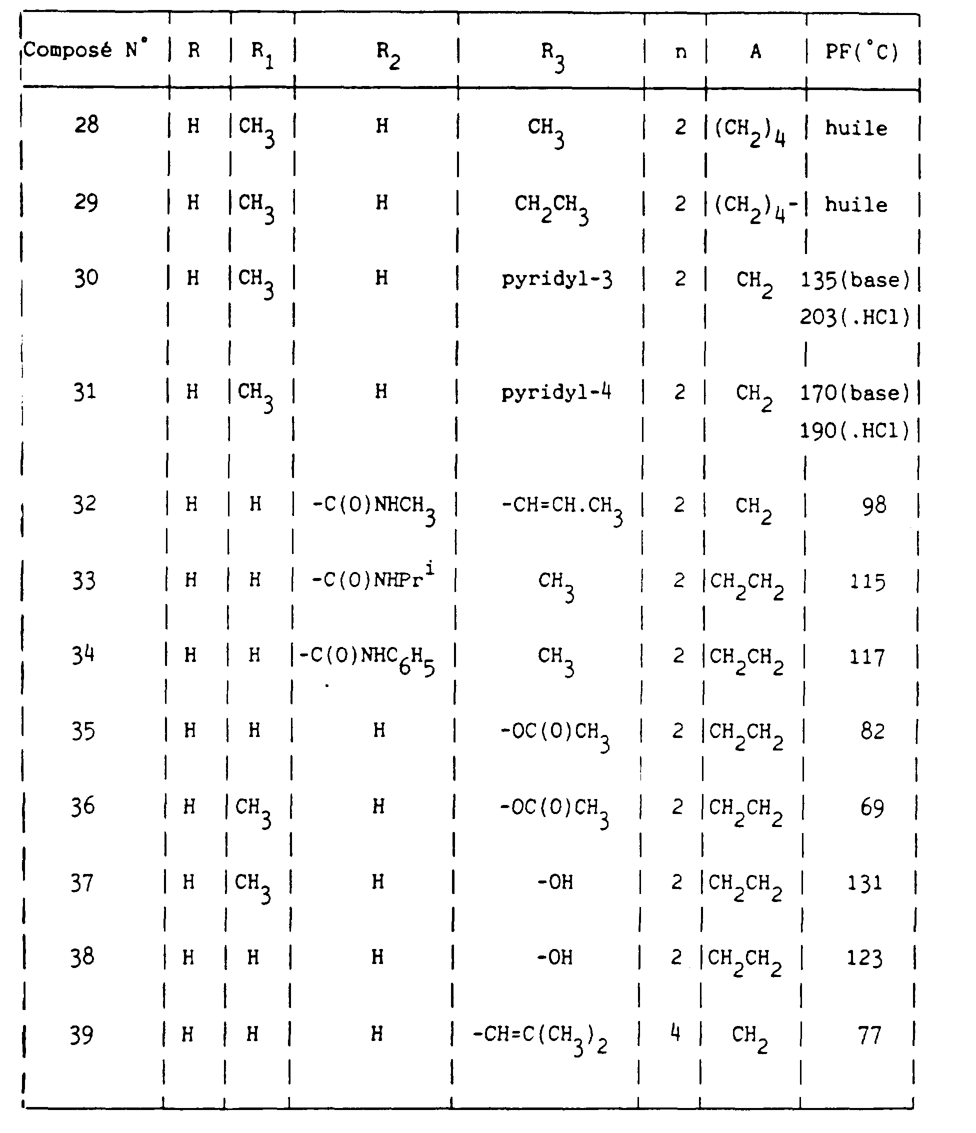

Les composés de l'invention ont été soumis à des essais pharmacologiques qui ont montré leur intérêt en tant que broncholytiques. A cet effet, les composés ont été étudiés pour leur effet antagoniste des contractions provoquées par divers produits selon le protocole suivant.

Un morceau de trachée de cobaye tricolore mâle de 400 g en moyenne est découpé en spirale et monté (2,5 cm environ) dans une cuve à organe isolé thermostatée à 37°C et remplie de tyrode oxygéné. On enregistre les contractions isométriques provoquées par les divers médiateurs grâce à un capteur UC2 Gould Statham (Gould Inc. Oxnard, California, USA) ou UF1 Palmer Bioscience relié à un enregistreur potentiométrique (Linseiss, Selb, RFA). Au début de l'expérience, le lambeau de trachée est soumis à une tension de 1 g et laissé au repos durant 1 h.

On utilise les concentrations suivantes de médiateurs (concentrations provoquant en général une contraction maximale) : dichlorhydrate d'histamine, 10 µg/ml ; chlorure d'acétylcholine, 1 µg/ml ; sel de potassium de leucotriene D₄, 0,05 µg/ml ; chlorure de potassium, 1850 µg/ml. Les concentrations d'agents utilisés entraînent des contractions qui deviennent maximales au bout de 5 à 15 mn suivant les médiateurs et se maintiennent en palier ensuite. Une concentration du produit étudié est administrée pour chaque contraction et le produit est laissé au contact de la trachée durant 5 mn. On mesure l'inhibition éventuelle obtenue au bout de ce temps (pourcentage de variation de l'amplitude de la contraction). La trachée est ensuite lavée durant 15 secondes et laissée au repos durant 10 mn environ (temps nécessaire au retour au tonus de base) avant de provoquer une nouvelle contraction.

Les produits insolubles dans l'eau sont dilués dans 0,178 % (v/v) (concentration finale dans le bain) de diméthyl sulfoxide (DMSO). Cette concentration de DMSO n'entraîne aucun effet vis-à-vis des contractions des divers médiateurs étudiés.

Les concentrations inhibitrices 50 % (CI 50) sont calculées en utilisant un programme SAS (Statistical Analysis System) inspiré de Bliss et Cattel (BLISS C.I. et CATTEL Mc K. Biological Assay Ann. Rev. Physiol. 5, 479, 1943).

Les résultats obtenus sur certains composés de l'invention sont reportés, à titre d'exemple, dans les tableaux 2 à 5.

**Tableau 2**

| Antagonisme de l'effet du chlorure de potassium | |
|---|---|
| composé n° | IC 50 (µg/ml) |
| 1 | 5,6 |
| 5 | 17 |
| 8 | 12,3 |
| 12 | 6,2 |
| théophylline | 21 |

**Tableau 3**

| Antagonisme de l'effet de l'histamine | |
|---|---|
| composé n° | IC 50 (µg/ml) |
| 1 | 0,9 |
| 3 | 3,3 |
| 11 | 3,45 |
| 12 | 0,54 |
| 17 | 0,43 |
| 19 | 1,13 |
| 20 | 1,55 |
| 24 | 1,3 |
| théophylline | 11,5 |

**Tableau 4**

| Antagonisme de l'effet de l'acétylcholine | |
|---|---|
| composé n° | IC 50 (µg/ml) |
| 1 | 25,5 |
| 3 | 7,1 |
| 12 | 15,5 |
| 19 | 16,5 |
| 20 | 9,7 |
| théophylline | 65 |

**Tableau 5**

| Antagonisme de l'effet du leucotriene D₄ | |
|---|---|
| composé n° | % d'inhibition à 10 µg/ml |
| 1 | - 71 |
| 12 | - 71 |
| 15 | - 64 |
| théophylline IC 50 = 13,5 µg/ml | |

Les composés de l'invention sont des broncholytiques qui peuvent être utilisés pour le traitement des maladies telles que les bronchopneumopathies obstructives chroniques, l'insuffisance respiratoire, l'emphysème, les pathologies cardiovasculaires relatives à l'insuffisance cardiaque.

Les compositions pharmaceutiques peuvent être sous forme appropriée pour l'administration par voie orale, rectale, parentérale ou locale, par exemple sous la forme de capsules, comprimés, granulés, gélules ou solutés liquides, sirops ou suspensions buvables, aérosols ou solutions pulvérisables, et contenir les excipients appropriés. La posologie quotidienne peut aller de 50 à 1 000 mg.

## Revendications (Revendications pour l'(les) Etat(s) contractant(s) suivant(s): AT, BE, CH, LI, DE, DK, FR, GB, IT, LU, NL, SE)

1. Nouveaux dérivés de dihydro-1,2 oxo-2 quinoxalines correspondant à la formule générale I : 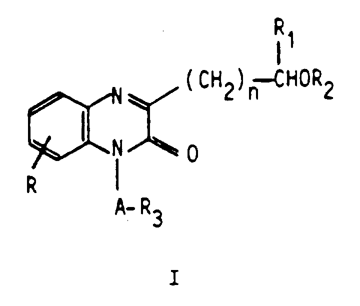 dans laquelle :
R représente un atome d'hydrogène ou d'halogène
R₁ représente un atome d'hydrogène ou un radical alkyle en C₁-C₄, droit ou ramifié
R₂ représente :
- un atome d'hydrogène
- un radical C(O)R₄
où R₄ est un groupe alkyle en C₁-C₄ droit ou ramifié
- un radical C(O)NHR₅
où R₅ est un groupe alkyle en C₁-C₄ droit ou ramifié, ou un groupe phényle
A représente une chaîne alkylène en C₁-C₄
R₃ représente :
- un atome d'hydrogène, un radical alkyle en C₁-C₄, un radical cycloalkyle en C₃-C₆, un groupe alcynyle, nitrile, hydroxyle, carboxamido, pyridyle, phényle, ou phényle substitué par un atome d'halogène, un groupe alkyle en C₁-C₄, un groupe alkoxy en C₁-C₄, ou un groupe nitro
- un radical alcényle de formule : 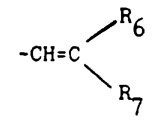 où R₆, R₇ peuvent être, indépendamment l'un de l'autre, un atome d'hydrogène, un groupe alkyle en C₁-C₄, un groupe phényle, ou un groupe alkoxy-carbonyle
- un radical de formule :
-OC(O)R₈
où R₈ représente un radical alkyle en C₁-C₄
- un radical de formule :
-OC(O)NHR₉
où R₉ représente un groupe alkyle en C₁-C₄, droit ou ramifié, ou un groupe phényle
n peut prendre des valeurs de 2 à 4
ainsi que les sels organiques ou minéraux thérapeutiquement acceptables de ces molécules.

2. Composés de formule générale I selon la revendication 1 caractérisés par le fait qu'ils sont choisis parmi :
- prényl-1 (hydroxy-3 propyl)-3 dihydro-1,2 oxo-2 quinoxaline
- allyl-1 (acétoxy-3 butyl)-3 dihydro-1,2 oxo-2 quinoxaline
- propyl-1 (méthylcarbamoyloxy-3 propyl)-3 dihydro-1,2 oxo-2 quinoxaline
- (méthylcarbamoyloxy-2 éthyl)-1 (hydroxy-3 butyl)-3 dihydro-1,2 oxo-2 quinoxaline
- allyl-1 (hydroxy-3 propyl)-3 chloro-7 dihydro-1,2 oxo-2 quinoxaline
- propargyl-1 (hydroxy-3 propyl)-3 chloro-6(7) dihydro-1,2 oxo-2 quinoxaline
- allyl-1 (hydroxy-4 butyl)-3 dihydro-1,2 oxo-2 quinoxaline
- allyl-1 (hydroxy-3 propyl)-3 dihydro-1,2 oxo-2 quinoxaline
- allyl-1 (hydroxy-3 butyl)-3 dihydro-1,2 oxo-2 quinoxaline
- (méthylcrotonyl)-1 (hydroxy-3 propyl)-3 dihydro-1,2 oxo-2 quinoxaline
- propyl-1 (hydroxy-3 propyl)-3 dihydro-1,2 oxo-2 quinoxaline
- crotyl-1 (hydroxy-3 propyl)-3 dihydro-1,2 oxo-2 quinoxaline
- propargyl-1 (hydroxy-3 propyl)-3 dihydro-1,2 oxo-2 quinoxaline
- cinnamyl-1 (hydroxy-3 propyl)-3 dihydro-1,2 oxo-2 quinoxaline
- cyclopropylméthyl-1 (hydroxy-3 propyl)-3 dihydro-1,2 oxo-2 quinoxaline
- cyanométhyl-1 (hydroxy-3 propyl)-3 dihydro-1,2 oxo-2 quinoxaline
- benzyl-1 (hydroxy-3 propyl)-3 dihydro-1,2 oxo-2 quinoxaline
- acétamido-1 (hydroxy-3 propyl)-3 dihydro-1,2 oxo-2 quinoxaline
- crotyl-1 (hydroxy-3 butyl)-3 dihydro-1,2 oxo-2 quinoxaline
- prényl-1 (hydroxy-3 butyl)-3 dihydro-1,2 oxo-2 quinoxaline
- cyclopropylméthyl-1 (hydroxy-3 butyl)-3 dihydro-1,2 oxo-2 quinoxaline
- (p.nitrobenzyl)-1 (hydroxy-3 propyl)-3 dihydro-1,2 oxo-2 quinoxaline
- propyl-1 (hydroxy-3 butyl)-3 dihydro-1,2 oxo-2 quinoxaline
- (p.méthoxybenzyl)-1 (hydroxy-3 butyl)-3 dihydro-1,2 oxo-2 quinoxaline
- méthyl-1 (hydroxy-3 butyl)-3 dihydro-1,2 oxo-2 quinoxaline
- butyl-1 (hydroxy-3 butyl)-3 dihydro-1,2 oxo-2 quinoxaline
- (pyridyl-2 méthyl)-1 (hydroxy-3 butyl)-3 dihydro-1,2 oxo-2 quinoxaline
- pentyl-1 (hydroxy-3 butyl)-3 dihydro-1,2 oxo-2 quinoxaline
- hexyl-1 (hydroxy-3 butyl)-3 dihydro-1,2 oxo-2 quinoxaline
- (pyridyl-3 méthyl)-1 (hydroxy-3 butyl)-3 dihydro-1,2 oxo-2 quinoxaline
- (pyridyl-4 méthyl)-1 (hydroxy-3 butyl)-3 dihydro-1,2 oxo-2 quinoxaline
- crotyl-1 (méthylcarbamoyloxy-3 propyl)-3 dihydro-1,2 oxo-2 quinoxaline
- propyl-1 (isopropylcarbamoyloxy-3 propyl)-3 dihydro-1,2 oxo-2 quinoxaline
- propyl-1 (phénylcarbamoyloxy-3 propyl)-3 dihydro-1,2 oxo-2 quinoxaline
- (acétoxy-2 éthyl)-1 (hydroxy-3 propyl)-3 dihydro-1,2 oxo-2 quinoxaline
- (acétoxy-2 éthyl)-1 (hydroxy-3 butyl)-3 dihydro-1,2 oxo-2 quinoxaline
- (hydroxy-2 éthyl)-1 (hydroxy-3 butyl)-3 dihydro-1,2 oxo-2 quinoxaline
- (hydroxy-2 éthyl)-1 (hydroxy-3 propyl)-3 dihydro-1,2 oxo-2 quinoxaline
- prényl-1 (hydroxy-5 pentyl)-3 dihydro-1,2 oxo-2 quinoxaline

3. Procédé de préparation de composés chimiques selon les revendications 1 et 2, caractérisé en ce que l'on obtient les composés III par réaction d'une orthophénylène diamine avec un céto-acide de formule II : 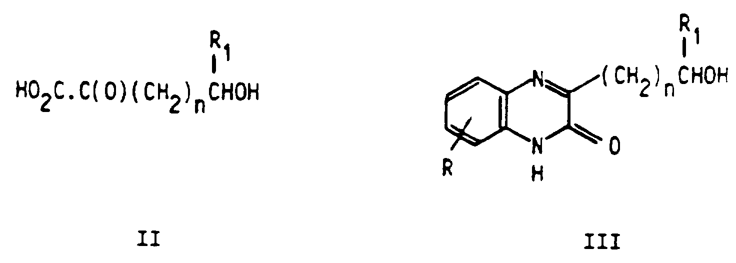 n, R, R₁ ayant la même signification que dans la revendication 1.

4. Procédé de préparation de composés chimiques selon les revendications 1, et 3 caractérisé en ce que la réaction des composés II avec l'orthophénylène diamine pour obtenir les composés de formule III s'effectue au sein d'un solvant alcoolique tel que l'éthanol, ou un solvant acide, tel que l'acide acétique, à une température allant de la température ambiante à la température du reflux du solvant.

5. Procédé de préparation de composés chimiques selon les revendications 1 et 2 caractérisé en ce que l'on transforme les composés de formule générale III en composés de formule générale IV 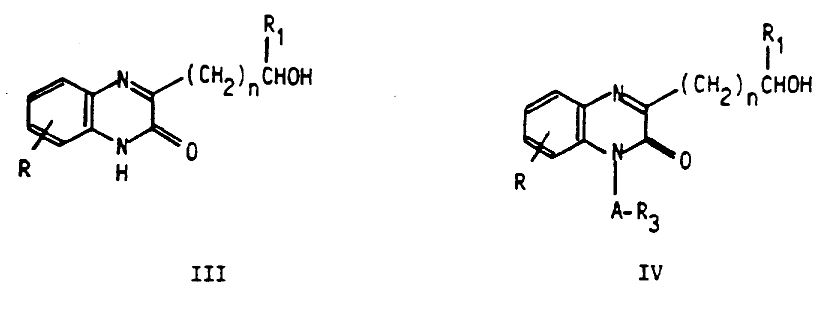 n, R, R₁, A et R₃ ayant la même signification que dans la revendication 1.

6. Procédé de préparation de composés chimiques selon les revendications 1, 2 et 3 caractérisé en ce que la transformation de composés de formule générale III en composés de formule générale IV se fait par réaction des composés III en présence d'une base tel que l'hydrure de sodium au sein d'un solvant aprotique tel que le DMF avec un réactif de formule R₃-A-X, où X est un atome d'iode, de brome, de chlore ou un groupe tel que mésylate ou tosylate.

7. Procédé de préparation de composés chimiques selon les revendications 1 et 2 caractérisé en ce que l'on fait réagir un composé de formule générale IV avec un anhydride de formule (R₄CO)₂O, de préférence à chaud, pour obtenir un composé de formule générale I où R₂ représente C(O)R₄, et où n, R, R₁, A, R₃ et R₄ ont la même signification que dans la revendication 1 :

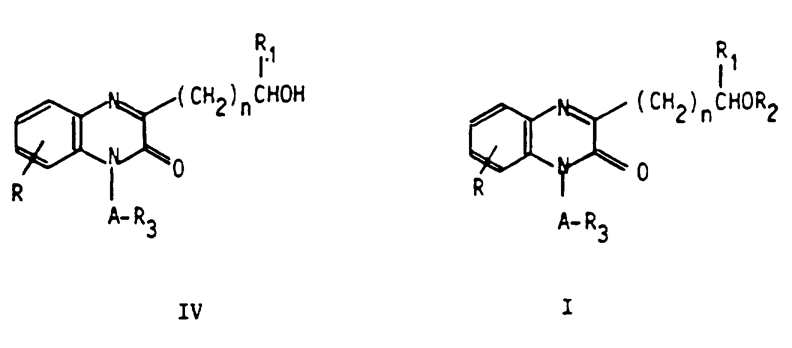

8. Procédé de préparation de composés chimiques selon les revendications 1 et 2 caractérisé en ce que :
- l'on fait réagir un composé de formule générale III avec un anhydride de formule (R₄CO)₂O, de préférence à chaud,
- l'on fait réagir le produit ainsi obtenu en présence d'une base tel que l'hydrure de sodium, avec un réactif de formule R₃-A-X, où X est un atome d'iode, de brome, de chlore, ou un groupe tel que mésylate ou tolylate pour obtenir un composé de formule générale I où R₂ représente C(O)R₄, et où n, R, R₁, A, R₃ et R₄ ont la même signification que dans la revendication 1 :

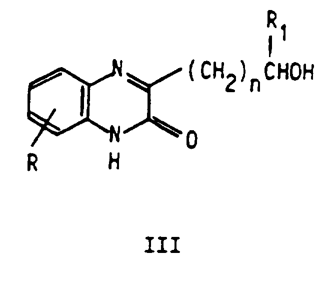

9. Procédé de préparation de composés chimiques selon les revendications 1, 2 et 3 caractérisé en ce que l'on fait réagir un composé de formule générale IV avec un isocyanate de formule R₅NCO dans un solvant aprotique tel que le toluène pour obtenir des composés de formule générale I où R₂ représente -C(O)NHR₅, et où n, R, R₁, A, R₃ et R₅ ont la même signification que dans la revendication 1.

10. Procédé de préparation de composés chimiques selon les revendications 1, 2 et 3 caractérisé en ce que l'on fait réagir un composé de formule générale IV avec le phosgène et ensuite avec une amine de formule R₅NH₂ pour obtenir des composés de formule générale I où R₂ représente -C(O)NHR₅, et où n, R, R₁, A, R₃ et R₅ ont la même signification que dans la revendication 1.

11. Procédé de préparation de composés chimiques selon les revendications 1 et 2 caractérisé en ce que l'on fait réagir un produit de formule générale I, où R₃ représente un radical hydroxyle, avec un excès d'anhydride de formule (R₈CO)₂O, de préférence à chaud, pour obtenir un composé de formule générale I où R₃ représente un groupe -OC(O)R₈, et où n, R, R₁, R₂, A et R₈ ont la même signification que dans la revendication 1.

12. Procédé de préparation de composés chimiques selon les revendications 1 et 2 caractérisé en ce que l'on fait réagir un produit de formule générale I, où R₃ représente un radical hydroxyle, avec un isocyanate de formule R₉NCO au sein d'un solvant aprotique tel que le toluène ou le xylène pour obtenir un produit de formule générale I où R₃ représente un radical -OC(O)NHR₉, et où n, R, R₁, R₂, A et R₉ ont la même signification que dans la revendication 1.

13. Médicaments pour le traitement des bronchopneumopathies obstructives chroniques, de l'insuffisance respiratoire, de l'emphysème, des pathologies cardiovasculaires relatives à l'insuffisance cardiaque, contenant les composés définis selon l'une des revendications 1 et 2.

14. Composition pharmaceutique caractérisée en ce qu'elle contient un composé de l'une des revendications 1 et 2.

15. Composition pharmaceutique caractérisée en ce qu'elle contient un composé de l'une des revendications 1 et 2 en association avec tout excipient approprié.

16. Composition pharmaceutique caractérisée en ce qu'elle contient un composé de l'une des revendications 1 et 2 associé à un autre principe actif.

## Revendications (Revendications pour l'(les) Etat(s) contractant(s) suivant(s): ES, GR)

1. Procédé de préparation de dérivés de dihydro-1,2 oxo-2 quinolaxines correspondant à la formule générale I : 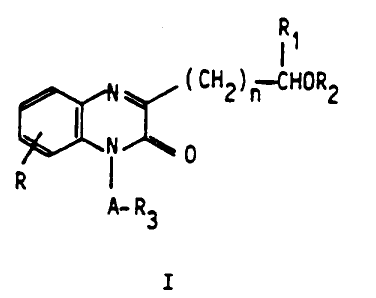 dans laquelle :
R représente un atome d'hydrogène ou d'halogène
R₁ représente un atome d'hydrogène ou un radical alkyle en C₁-C₄, droit ou ramifié
R₂ représente :
- un atome d'hydrogène
- un radical C(O)R₄
où R₄ est un groupe alkyle en C₁-C₄ droit ou ramifié
- un radical C(O)NHR₅
où R₅ est un groupe alkyle en C₁-C₄ droit ou ramifié, ou un groupe phényle
A représente une chaîne alkylène en C₁-c₄
R₃ représente :
- un atome d'hydrogène, un radical alkyle en C₁-C₄, un radical cycloalkyle en C₃-C₆, un groupe alcynyle, nitrile, hydroxyle, carboxamido, pyridyle, phényle, ou phényle substitué par un atome d'halogène, un groupe alkyle en C₁-C₄, un groupe alkoxy en C₁-C₄, ou un groupe nitro
- un radical alcényle de formule : 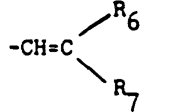 où R₆, R₇ peuvent être, indépendamment l'un de l'autre, un atome d'hydrogène, un groupe alkyle en C₁-C₄, un groupe phényle, ou un groupe alkoxycarbonyle
- un radical de formule :
-OC(O)R₈
où R₈ représente un radical alkyle en C₁-C₄
- un radical de formule :
-OC(O)NHR₉
où R₉ représente un groupe alkyle en C₁-C₄, droit ou ramifié, ou un groupe phényle
n peut prendre des valeurs de 2 à 4
ainsi que les sels organiques ou minéraux thérapeutiquement acceptables de ces molécules.

2. Procédé de préparation des composés de formule générale I selon la revendication 1 caractérisé par le fait que les composés sont choisis parmi :
- prényl-1 (hydroxy-3 propyl)-3 dihydro-1,2 oxo-2 quinoxaline
- allyl-1 (acétoxy-3 butyl)-3 dihydro-1,2 oxo-2 quinoxaline
- propyl-1 (méthylcarbamoyloxy-3 propyl)-3 dihydro-1,2 oxo-2 quinoxaline
- (methylcarbamoyloxy-2 éthyl)-1 (hydroxy-3 butyl)-3 dihydro-1,2 oxo-2 quinoxaline
- allyl-1 (hydroxy-3 propyl)-3 chloro-7 dihydro-1,2 oxo-2 quinoxaline
- propargyl-1 (hydroxy-3 propyl)-3 chloro-6(7) dihydro-1,2 oxo-2 quinoxaline
- allyl-1 (hydroxy-4 butyl)-3 dihydro-1,2 oxo-2 quinoxaline
- allyl-1 (hydroxy-3 propyl)-3 dihydro-1,2 oxo-2 quinoxaline
- allyl-1 (hydroxy-3 butyl)-3 dihydro-1,2 oxo-2 quinoxaline
- (méthylcrotonyl)-1 (hydroxy-3 propyl)-3 dihydro-1,2 oxo-2 quinoxaline
- propyl-1 (hydroxy-3 propyl)-3 dihydro-1,2 oxo-2 quinoxaline
- crotyl-1 (hydroxy-3 propyl)-3 dihydro-1,2 oxo-2 quinoxaline
- propargyl-1 (hydroxy-3 propyl)-3 dihydro-1,2 oxo-2 quinoxaline
- cinnamyl-1 (hydroxy-3 propyl)-3 dihydro-1,2 oxo-2 quinoxaline
- cyclopropylméthyl-1 (hydroxy-3 propyl)-3 dihydro-1,2 oxo-2 quinoxaline
- cyanométhyl-1 (hydroxy-3 propyl)-3 dihydro-1,2 oxo-2 quinoxaline
- benzyl-1 (hydroxy-3 propyl)-3 dihydro-1,2 oxo-2 quinoxaline
- acétamido-1 (hydroxy-3 propyl)-3 dihydro-1,2 oxo-2 quinoxaline
- crotyl-1 (hydroxy-3 butyl)-3 dihydro-1,2 oxo-2 quinoxaline
- prényl-1 (hydroxy-3 butyl)-3 dihydro-1,2 oxo-2 quinoxaline
- cyclopropylméthyl-1 (hydroxy-3 butyl)-3 dihydro-1,2 oxo-2 quinoxaline
- (p.nitrobenzyl)-1 (hydroxy-3 propyl)-3 dihydro-1,2 oxo-2 quinoxaline
- propyl-1 (hydroxy-3 butyl)-3 dihydro-1,2 oxo-2 quinoxaline
- (p.méthoxybenzyl)-1 (hydroxy-3 butyl)-3 dihydro-1,2 oxo-2 quinoxaline
- méthyl-1 (hydroxy-3 butyl)-3 dihydro-1,2 oxo-2 quinoxaline
- butyl-1 (hydroxy-3 butyl)-3 dihydro-1,2 oxo-2 quinoxaline
- (pyridyl-2 méthyl)-1 (hydroxy-3 butyl)-3 dihydro-1,2 oxo-2 quinoxaline
- pentyl-1 (hydroxy-3 butyl)-3 dihydro-1,2 oxo-2 quinoxaline
- hexyl-1 (hydroxy-3 butyl)-3 dihydro-1,2 oxo-2 quinoxaline
- (pyridyl-3 méthyl)-1 (hydroxy-3 butyl)-3 dihydro-1,2 oxo-2 quinoxaline
- (pyridyl-4 méthyl)-1 (hydroxy-3 butyl)-3 dihydro-1,2 oxo-2 quinoxaline
- crotyl-1 (methylcarbamoyloxy-3 propyl)-3 dihydro-1,2 oxo-2 quinoxaline
- propyl-1 (isopropylcarbamoyloxy-3 propyl)-3 dihydro-1,2 oxo-2 quinoxaline
- propyl-1 (phénylcarbamoyloxy-3 propyl)-3 dihydro-1,2 oxo-2 quinoxaline
- (acétoxy-2 éthyl)-1 (hydroxy-3 propyl)-3 dihydro-1,2 oxo-2 quinoxaline
- (acétoxy-2 éthyl)-1 (hydroxy-3 butyl)-3 dihydro-1,2 oxo-2 quinoxaline
- (hydroxy-2 éthyl)-1 (hydroxy-3 butyl)-3 dihydro-1,2 oxo-2 quinoxaline
- (hydroxy-2 éthyl)-1 (hydroxy-3 propyl)-3 dihydro-1,2 oxo-2 quinoxaline
- prényl-1 (hydroxy-5 pentyl)-3 dihydro-1,2 oxo-2 quinoxaline

3. Procédé de préparation de composés chimiques selon les revendications 1 et 2, caractérisé en ce que l'on obtient les composés III par réaction d'une orthophénylène diamine avec un céto-acide de formule II : 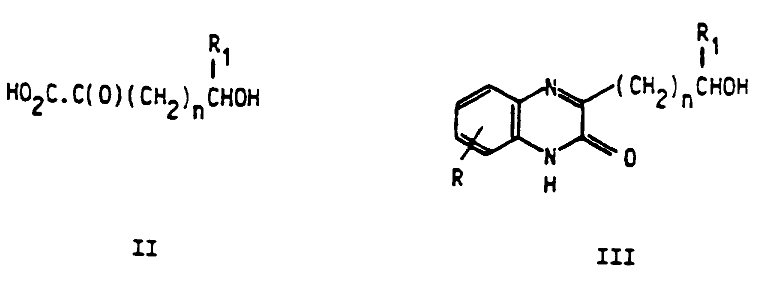 n, R, R₁ ayant la même signification que dans la revendication 1.

4. Procédé de préparation de composés chimiques selon les revendications 1, 2 et 3 caractérisé en ce que la réaction des composés II avec l'orthophényléne diamine pour obtenir les composes de formule III s'effectue au sein d'un solvant alcoolique tel que l'éthanol, ou un solvant acide, tel que l'acide acétique, à une température allant de la température ambiante à la température du reflux du solvant.

5. Procédé de préparation de composés chimiques selon les revendications 1 et 2 caractérisé en ce que l'on transforme les composés de formule générale III en composés de formule générale IV 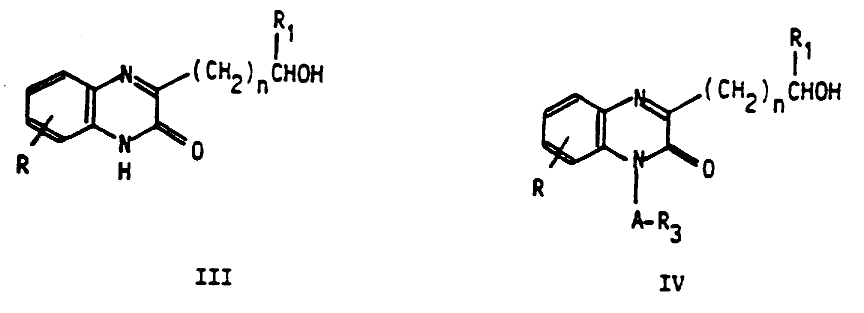 n, R, R₁, A et R₃ ayant la même signification que dans la revendication 1.

6. Procédé de préparation de composés chimiques selon les revendications 1, 2 et 3 caractérisé en ce que la transformation de composés de formule générale III en composés de formule générale IV se fait par réaction des composés III en présence d'une base tel que l'hydrure de sodium au sein d'un solvant aprotique tel que le DMF avec un réactif de formule R₃-A-X, où X est un atome d'iode, de brome, de chlore ou un groupe tel que mésylate ou tosylate.

7. Procédé de préparation de composés chimiques selon les revendications 1 et 2 caractérisé en ce que l'on fait réagir un composé de formule générale IV avec un anhydride de formule (R₄CO)₂O, de préférence à chaud, pour obtenir un composé de formule générale I où R₂ représente C(O)R₄, et où n, R, R₁, A, R₃ et R₄ ont la même signification que dans la revendication 1 :

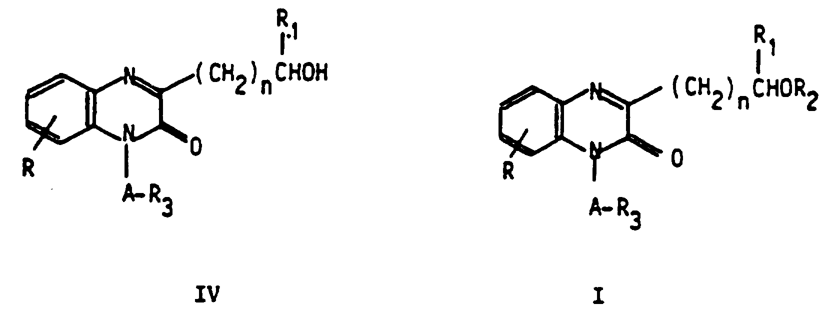

8. Procédé de préparation de composés chimiques selon les revendications 1 et 2 caractérisé en ce que :
- l'on fait réagir un composé de formule générale III avec un anhydride de formule (R₄CO)₂O, de préférence à chaud,
- l'on fait réagir le produit ainsi obtenu en présence d'une base tel que l'hydrure de sodium, avec un réactif de formule R₃-A-X, où X est un atome d'iode, de brome, de chlore, ou un groupe tel que mésylate ou tolylate pour obtenir un composé de formule générale I où R₂ représente C(O)R₄, et où n, R, R₁, A, R₃ et R₄ ont la même signification que dans la revendication 1 :

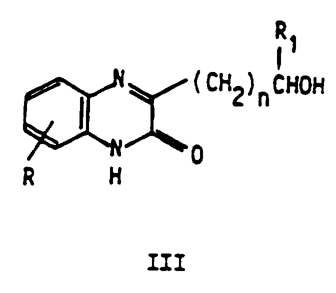

9. Procédé de préparation de composés chimiques selon les revendications 1, 2 et 3 caractérisé en ce que l'on fait réagir un composé de formule générale IV avec un isocyanate de formule R₅NCO dans un solvant aprotique tel que le toluène pour obtenir des composés de formule générale I où R₂ représente -C(O)NHR₅, et où n, R, R₁, A, R₃ et R₅ ont la même signification que dans la revendication 1.

10. Procédé de préparation de composés chimiques selon les revendications 1, 2 et 3 caractérisé en ce que l'on fait réagir un composé de formule générale IV avec le phosgéne et ensuite avec une amine de formule R₅NH₂ pour obtenir des composés de formule générale I où R₂ représente -C(O)NHR₅, et où n, R, R₁, A, R₃ et R₅ ont la même signification que dans la revendication 1.

11. Procédé de préparation de composés chimiques selon les revendications 1 et 2 caractérisé en ce que l'on fait réagir un produit de formule générale I, où R₃ représente un radical hydroxyle, avec un excès d'anhydride de formule (R₈CO)₂O, de préférence à chaud, pour obtenir un composé de formule générale I où R₃ représente un groupe -OC(O)R₈, et où n, R, R₁, R₂, A et R₈ ont la même signification que dans la revendication 1.

12. Procédé de préparation de composés chimiques selon les revendications 1 et 2 caractérisé en ce que l'on fait réagir un produit de formule générale I, où R₃ représente un radical hydroxyle, avec un isocyanate de formule R₉NCO au sein d'un solvant aprotique tel que le toluène ou le xylène pour obtenir un produit de formule générale I où R₃ représente un radical -OC(O)NHR₉, et où n, R, R₁, R₂, A et R₉ ont la même signification que dans la revendication 1.

13. Procédé de préparation de composés chimiques selon les revendications 1 et 2 caractérisé en ce que les composés obtenus sont utiles dans la préparation de médicaments.

## Patentansprüche (Patentansprüche für folgende(n) Vertragsstaat(en): AT, BE, CH, LI, DE, DK, FR, GB, IT, LU, NL, SE)

1. Neue Derivate von Dihydro-1,2 Oxy-2 Chinoxalinen nach der allgemeinen Formel I: 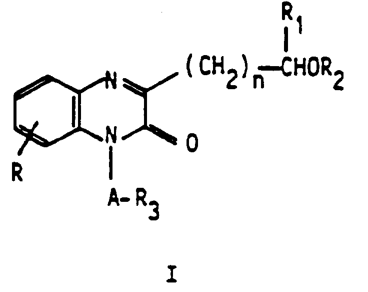 worin:
R ein Wasserstoff- oder Halogenatom darstellt
R₁ ein Wasserstoffatom oder ein Alkylradikal mit direkter oder verzweigter Bindung in C₁-C₄ darstellt
R₂ ist:
- ein Wasserstoffatom
- ein C(O)R₄ Radikal
wobei R₄ ein direkte oder verzweigte Alkylgruppe in C₁-C₄ ist
- ein C(O)NHR₅ Radikal
wobei R₅ eine direkte oder verzweigte Alkylgruppe in C₁-C₄ oder eine Phenylgruppe ist
A stellt eine Alkylenkette in C₁-C₄ dar
R₃ ist:
- ein Wasserstoffatom, ein Alkylradikal, in C₁-C₄, ein Cycloalkylradikal in C₃-C₆, eine Alkynyl-, Nitryl-, Hydroxyl, Carboxamid-; Pyridyl-, Phenylgruppe oder Phenyl, an dessen Stelle ein Halogenatom tritt, eine Alkylgruppe in C₁-C₄, eine Alkoxygruppe in C₁-C₄, oder eine Nitrogruppe.
- ein Alkenylradikal mit der Formel: 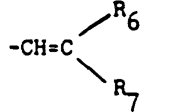 in welcher R₆, R₇ unabhängig voneinander ein Wasserstoffatom, eine Alkylgruppe in C₁-C₄, eine Phenylgruppe, oder eine Alkoxy-Carbonylgruppe sein können,
- ein Radikal mit der Formel:
-OC(O)R₈
worin R₈ ein Alkylradikal in C₁-C₄ darstellt,
- ein Radikal mit der Formel:
-OC(O)NHR₉
worin R₉ ein direkte oder verzweigte Alkylgruppe bzw. eine Phenylgruppe in C₁-C₄ darstellt.
n kann Werte von 2 bis 4 annehmen.
Weiterhin organische oder mineralisch Salze als therapeutisch akzeptable Moleküle derselben.

2. Verbindungen der allgemeinen Formel I nach Patentanspruch 1, gekennzeichnet dadurch, daß sie aus folgenden ausgewählt wurden:
- Prenyl-1 (Hydroxy-3 Propyl)-3 Dihydro-1,2 Oxy-2 Chinoxalin
- Allyl-1 (Acetoxy-3 Butyl)-3 Dihydro-1,2 Oxy-2 Chinoxalin
- Propyl 1 (methylcarbamoyloxy-3 Propyl)-3 Dihydro-1,2 Oxy-2 Chinoxalin
- (Methylcarbamoyloxy-2 ethyl)-1 (Hydroxy-3 butyl)-3 Dihydro-1,2 Oxy-2 Chinoxalin
- Allyl-1 (Hydroxy-3 Propyl)-3 Chloro-7 Dihydro-1,2 Oxy-2 Chinoxalin
- Propargyl-1 (Hydroxy-3 Propyl)-3 Chloro-6(7) Dihydro-1,2 Oxy-2 Chinoxalin
- Allyl-1 (Hydroxy-4 Butyl)-3 Dihydro-1,2 Oxy-2 Chinoxalin
- Allyl-1 (Hydroxy-3 Propyl)-3 Dihydro-1,2 Oxy-2 Chinoxalin
- Allyl-1 (Hydroxy-3 Butyl)-3 Dihydro-1,2 Oxy-2 Chinoxalin
- (Methylcrotonyl)-1 (Hydroxy-3 Propyl)-3 Dihydro- 1,2 Oxy-2 Chinoxalin
- Propyl-1 (Hydroxy-3 Propyl)-3 Dihydro-1,2 Oxy-2 Chinoxalin
- Crotyl-1 (Hydroxy-3 Propyl)-3 Dihydro-1,2 Oxy-2 Chinoxalin
- Propargyl-1 (Hydroxy-3 Propyl)-3 Dihydro-1,2 Oxy-2 Chinoxalin
- Cinnamyl- (Hydroxy-3 Propyl)-3 Dihydro-1,2 Oxy-2 Chinoxalin
- Cyclopropylmethyl-1 (Hydroxy-3 Propyl)-3 Dihydro-1,2 Oxy-2 Chinoxalin
- Cyanomethyl-1 (Hydroxy-3 Propyl)-3 Dihydro-1,2 Oxy-2 Chinoxalin
- Benzyl-1 (Hydroxy-3 Propyl)-3 Dihydro-1,2 Oxy-2 Chinoxalin
- Acetamido-1 (Hydroxy-3 Propyl)-3 Dihydro-1,2 Oxy-2 Chinoxalin
- Crotyl-1 (Hydroxy-3 butyl)-3 Dihydro-1,2 Oxy-2 Chinoxalin
- Prenyl-1 (Hydroxy-3 butyl)-3 Dihydro-1,2 Oxy-2 Chinoxalin
- Cyclopropylmethyl-1 (Hydroxy-3 Butyl)-3 Dihydro-1,2 Oxy-2 Chinoxalin
- (P. nitrobenzyl)-1 (Hydroxy-3 Propyl)-3 Dihydro-1,2 Oxy-2 Chinoxalin
- Propyl-1 (Hydroxy-3 butyl)-3 Dihydro-1,2 Oxy-2 Chinoxalin
- (P. methoxybenzyl)-1 (Hydroxy-3 butyl)-3 Dihydro-1,2 Oxy-2 Chinoxalin
- Methyl-1 (Hydroxy-3 Butyl)-3 Dihydro-1,2 Oxy-2 Chinoxalin
- Butyl-1 (Hydroxy-3 Butyl)-3 Dihydro-1,2 Oxy-2 Chinoxalin
- (Pyridyl-2 Methyl)-1 (Hydroxy-3 butyl)-3 Dihydro-1,2 Oxy-2 Chinoxalin
- Pentyl-1 (Hydroxy-3 Butyl)-3 Dihydro-1,2 Oxy-2 Chinoxalin
- Hexyl-1 (Hydroxy-3 butyl)-3 Dihydro-1,2 Oxy-2 Chinoxalin
- (Pyridyl-3 Methyl)-1 (Hydroxy-3 Butyl)-3 Dihydro-1,2 Oxy-2 Chinoxalin
- (Pyridyl-4 Methyl)-1 (Hydroxy-3 Butyl)-3 Dihydro-1,2 Oxy-2 Chinoxalin
- Crotyl-1 (Methylcarbamoyloxy-3 Propyl)-3 Dihydro-1,2 Oxy-2 Chinoxalin
- Propyl-1 (Isopropylcarbamoyloxy-3 Propyl)-3 Dihydro-1,2 Oxy-2 Chinoxalin
- Propyl-1 (Phenylcarbamoyloxy-3 Propyl)-3 Dihydro-1,2 Oxy-2 Chinoxalin
- (Acetoxy-2 Ethyl)-1 (Hydroxy-3 Propyl)-3 Dihydro-1,2 Oxy-2 Chinoxalin
- (Acetoxy-2 Ethyl)-1 (Hydroxy-3 butyl)-3 Dihydro-1,2 Oxy-2 Chinoxalin
- (Hydroxy-2 Ethyl)-1 (Hydroxy-3 Butyl)-3 Dihydro-1,2 Oxy-2 Chinoxalin
- (Hydroxy-2 ethyl)-1 (Hydroxy-3 Propyl)-3 Dihydro-1,2 Oxy-2 Chinoxalin
- Prenyl-1 (Hydroxy-5 Pentyl)-3 Dihydro-1,2 Oxy-2 Chinoxalin

3. Zubereitungsprozedur für chemische Verbindungen nach Patentanspruch 1 und 2, gekennzeichnet durch die Tatsache, daß die Verbindungen III durch Reaktion eines Orthophenylen-Diamin mit Acetosäure der Formel II gewonnen werden:
Formel II: 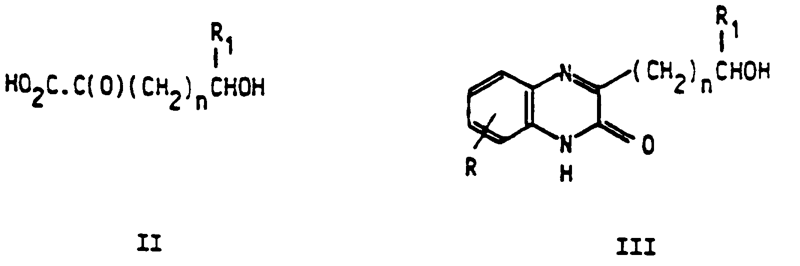 wobei n, R und R1 die gleiche Bedeutung wie in Patentanspruch 1 haben.

4. Zubereitungsprozedur für chemische Verbindungen nach Patentanspruch 1, 2 und 3, gekennzeichnet durch die Tatsache, daß die Reaktion der Verbindungen II mit Orthophylen-Diamin zur Herstellung von Verbindungen III in einer alkoholischen Lösung wie z.B. Äthanol, oder in einer sauren Lösung wie z.B. Ameisensäure gewonnen werden, wobei die Temperatur von Umgebungstemperatur bis zur Temperatur des Rückflusses des Lösungsmittels reichen kann.

5. Zubereitungsprozedur für chemische Verbindungen nach Patentanspruch 1 und 2, gekennzeichnet durch die Tatsache, daß die Verbindungen der allgemeinen Formel III in Verbindungen der allgemeinen Formel IV umgewandelt werden. 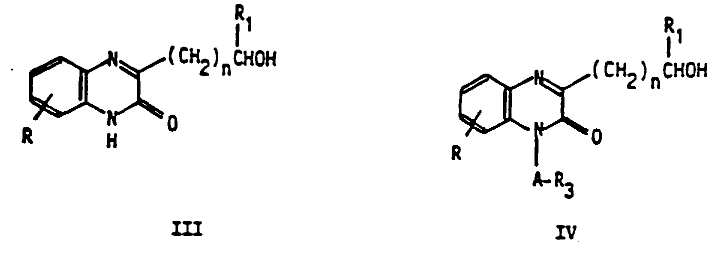 wobei n, R, R₁, A und R₃ die gleiche Bedeuting wie in Patentanspruch 1 haben.

6. Zubereitungsprozedur für chemische Verbindungen nach Patentanspruch 1, 2 und 3, gekennzeichnet durch die Tatsache, daß die Umwandlung von Verbindungen nach der allgemeinen Formel III in Verbindungen der allgemeinen Formel IV durch Reaktion der III-Verbindungen in Gegenwart von einer Base wie z.B. N atriumhydrid in einem aprotischen Lösungsmittel wie DMF mit einem Reagenz der Formel R₃-A-X gewonnen werden, worin X ein Atom von Jod, Brom, Chlor oder eine Gruppe wie Mesylat oder Tosylat ist.

7. Zubereitungsprozedur für chemische Verbindungen nach Patentanspruch 1 und 2, gekennzeichnet durch die Tatsache, daß man eine Verbindung der allgemeinen Formel IV mit eine Anhydrid der Formel (R₄CO)₂O reagieren läßt, und zwar vorzugsweise bei hoher Temperatur, um eine Verbindung der allgemeinen Formel I zu erhalten, worin R₂ für C(O)R₄ steht und n, R, R₁, A, R₃ und R₄ die gleiche Bedeutung wie in Patentanspruch 1 haben:

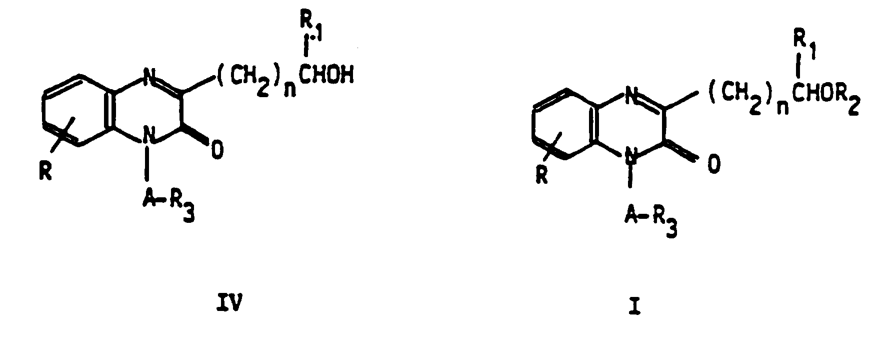

8. Zubereitungsprozedur für chemische Verbindungen nach Patentanspruch 1 und 2, gekennzeichnet durch:
- daß man eine Verbindung der allgemeinen Formel III mit einem Anhydrid der (R₄CO)₂O vorzugsweise bei hoher Temperatur reagieren läßt,
- daß man das derart erhaltene Reaktionsprodukt in Präsenz einer Base wie z.B. Natriumhydrid mit einem Reagenz der Formel R₃-A-X reagieren läßt, worin X ein Atom von Jod, Brom, Chlor oder eine Gruppe wie Mesylat oder Tosylat ist, um eine Verbindung der allgemeinen Formel I zu erhalten, in welcher R₂ für C(O)R₄ steht und n, R, R₁, A, R₃ sowie R₄ die gleiche Bedeutung haben wie in Patentanspruch 1:

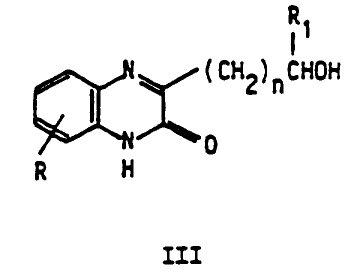

9. Zubereitungsprozedur für chemische Verbindungen nach Patentanspruch 1, 2 und 3, gekennzeichnet durch die Tatsache, daß man eine Verbindung der allgemeinen Formel IV mit Isocyanat der Formel R₅NCO in einem aprotischen Lösungsmittel wie Toluol reagieren läßt, um Verbindungen mit der allgemeinen Formel I zu erhalten, worin R₂ für -C(O)NHR₅ steht und n, R, R₁, A, R₃ sowie R₅ die gleiche Bedeutung haben wie in Patentanspruch 1.

10. Zubereitungsprozedur für chemische Verbindungen nach Patentanspruch 1, 2 und 3, gekennzeichnet durch die Tatsache, daß man eine Verbindung der allgemeinen Formel IV mit Phosgen, und dann mit einem Amin der Formel R₅NH₂ reagieren läßt, um Verbindungen mit der allgemeinen Formel I zu erhalten, worin R₂ für -C(O)R₅ steht und n, R, R₁, A, R₃ sowie R₅ die gleiche Bedeutung haben wie in Patentanspruch 1.

11. Zubereitungsprozedur für chemische Verbindungen nach Patentanspruch 1 und 2, gekennzeichnet durch die Tatsache, daß man eine Verbindung der allgemeinen Formel I, worin R₃ ein Hydroxylradikal ist, mit einem Überschuß der Formel (R₈CO)₂O reagieren läßt, und zwar vorzugsweise bei hohen Temperaturen, um eine Verbindung der allgemeinen Formel I zu erhalten, worin R₃ für eine -OC(O)R₈ -Gruppe steht und n, R, R₁, R₂, A sowie R₈ die gleiche Bedeutung haben wie in Patentanspruch 1.

12. Zubereitungsprozedur für chemische Verbindungen nach Patentanspruch 1 und 2, gekennzeichnet durch die Tatsache, daß man eine Verbindung der allgemeinen Formel I, worin R₃ ein Hydroxylradikal ist, mit einem Isocyanat der Formel R₉NCO in einem aprotischen Lösungsmittel wie Toluol oder Xylol reagieren läßt, um Verbindungen mit der allgemeinen Formel I zu erhalten, worin R₃ für -OC(O)NHR₉ steht und n, R, R₁, R₂, A sowie R₉ die gleiche Bedeutung haben wie in Patentanspruch 1.

13. Medikamtente für die Behandlung chronisch obstruktiver Bronchopneumopathies, Atmungsinsuffizienz, Emphysem sowie kardiovaskuläre Pathologien im Zusammenhang mit Herzfunktionsstörungen, welche Verbindungen nach einem der Patentansprüche 1 oder 2 enthalten.

14. Pharmazeutische Präparationen gekennzeichnet durch die Tatsache, daß sie eine der Verbindungen nach Patentanspruch 1 oder 2 enthalten.

15. Pharmazeutische Präparationen gekennzeichnet durch die Tatsache, daß sie eine der Verbindungen nach Patentanspruch 1 oder 2 zusammen mit einem angemessenen Exzipienten enthalten.

16. Pharmazeutische Präparationen gekennzeichnet durch die Tatsache, daß sie eine der Verbindungen nach Patentanspruch 1 oder 2 zusammen mit einem anderen Wirkstoff enthalten.

## Patentansprüche (Patentansprüche für folgende(n) Vertragsstaat(en): ES, GR)

1. Zubereitungsprozedur für Derivate von Dihydro-1,2 Oxy-2 Chinolaxinen nach der allgemeinen Formel I: 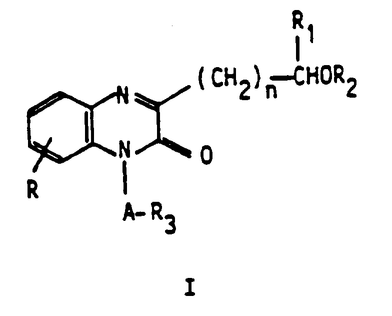 worin:
R ein Wasserstoff- oder Halogenatom darstellt
R₁ ein Wasserstoffatom oder ein Alkylradikal mit direkter oder verzweigter Bindung in C₁-C₄ darstellt
R₂ ist:
- ein Wasserstoffatom
- ein C(O)R₄ Radikal
wobei R₄ ein direkte oder verzweigte Alkylgruppe in C₁-C₄ ist
- ein C(O)NHR₅ Radikal
wobei R₅ eine direkte oder verzweigte Alkylgruppe in C₁-C₄ oder eine Phenylgruppe ist
A stellt eine Alkylenkette in C₁-C₄ dar
R₃ ist:
- ein Wasserstoffatom, ein Alkylradikal, in C₁-C₄, ein Cycloalkylradikal in C₃-C₆, eine Alkynyl-, Nitryl-, Hydroxyl, Carboxamid-, Pyridyl-, Phenylgruppe oder Phenyl, an dessen Stelle ein Halogenatom tritt, eine Alkylgruppe in C₁-C₄, eine Alkoxygruppe in C₁-C₄, oder eine Nitrogruppe.
- ein Alkenylenradikal mit der Formel: 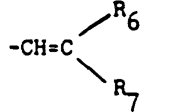 in welcher R₆, R₇ unabhängig voneinander ein Wasserstoffatom, eine Alkylgruppe in C₁-C₄, eine Phenylgruppe, oder eine Alkoxy-Carbonylgruppe sein können,
- ein Radikal mit der Formel:
-OC(O)R₈
worin R₈ ein Alkylradikal in C₁-C₄ darstellt,
- ein Radikal mit der Formel:
-OC(O)NHR₉
worin R₉ ein direkte oder verzweigte Alkylgruppe bzw. eine Phenylgruppe in C₁-C₄ darstellt.
n kann Werte von 2 bis 4 annehmen.
Weiterhin organische oder mineralische Salze als therapeutisch akzeptable Moleküle derselben.

2. Zubereitungsprozedur für Verbindungen der allgemeinen Formel I nach Patentanspruch 1 , die dadurch gekennzeichnet sind, daß die Verbindungen aus folgenden gewählt sind:
- Prenyl-1 (Hydroxy-3 Propyl)-3 Dihydro-1,2 Oxy-2 Chinoxalin
- Allyl-1 (Acetoxy-3 Butyl)-3 Dihydro-1,2 Oxy-2 Chinoxalin
- Propyl-1 (Methylcarbamoyloxy-3 Propyl)-3 Dihydro-1,2 Oxy-2 Chinoxalin
- (Methylcarbamoyloxy-2 Ethyl)-1 (Hydroxy-3 Butyl)-3 Dihydro-1,2 Oxy-2 Chinoxalin
- Allyl-1 (Hydroxy-3 Propyl)-3 Chloro-7 Dihydro-1,2 Oxy-2 Chinoxalin
- Propargyl-1 (Hydroxy-3 Propyl)-3 Chloro-6(7) Dihydro-1,2 Oxy-2 Chinoxalin
- Allyl-1 (Hydroxy-4 Butyl)-3 Dihydro-1,2 Oxy-2 Chinoxalin
- Allyl-1 (Hydroxy-3 Propyl)-3 Dihydro-1,2 Oxy-2 Chinoxalin
- Allyl-1 (Hydroxy-3 Butyl)-3 Dihydro-1,2 Oxy-2 Chinoxalin
- (Methylcrotonyl)-1 (Hydroxy-3 Propyl)-3 Dihydro-1,2 Oxy-2 Chinoxalin
- Propyl-1 (Hydroxy-3 Propyl)-3 Dihydro-1,2 Oxy-2 Chinoxalin
- Crotyl-1 (Hydroxy-3 Propyl)-3 Dihydro-1,2 Oxy-2 Chinoxalin
- Propargyl-1 (Hydroxy-3 Propyl)-3 Dihydro-1,2 Oxy-2 Chinoxalin
- Cinnamyl-1 (Hydroxy-3 Propyl)-3 Dihydro-1,2 Oxy-2 Chinoxalin
- Cyclopropylmethyl-1 (Hydroxy-3 Propyl)-3 Dihydro-1,2 Oxy-2 Chinoxalin
- Cyanomethyl-1 (Hydroxy-3 Propyl)-3 Dihydro-1,2 Oxy-2 Chinoxalin
- Benzyl-1 (Hydroxy-3 Propyl)-3 Dihydro-1,2 Oxy-2 Chinoxalin
- Acetamido-1 (Hydroxy-3 Propyl)-3 Dihydro-1,2 Oxy-2 Chinoxalin
- Crotyl-1 (Hydroxy-3 butyl)-3 Dihydro-1,2 Oxy-2 Chinoxalin
- Prenyl-1 (Hydroxy-3 butyl)-3 Dihydro-1,2 Oxy-2 Chinoxalin
- Cyclopropylmethyl-1 (Hydroxy-3 Butyl)-3 Dihydro-1,2 Oxy-2 Chinoxalin
- (P.nitrobenzyl)-1 (Hydroxy-3 Propyl)-3 Dihydro-1,2 Oxy-2 Chinoxalin
- Propyl-1 (Hydroxy-3 butyl)-3 Dihydro-1,2 Oxy-2 Chinoxalin
- (P. methoxybenzyl)-1 (Hydroxy-3 butyl)-3 Dihydro-1,2 Oxy-2 Chinoxalin
- Methyl-1 (Hydroxy-3 Butyl)-3 Dihydro-1,2 Oxy-2 Chinoxalin
- Butyl-1 (Hydroxy-3 Butyl)-3 Dihydro-1,2 Oxy-2 Chinoxalin
- (Pyridyl-2 Methyl)-1 (Hydroxy-3 butyl)-3 Dihydro-1,2 Oxy-2 Chinoxalin
- Pentyl-1 (Hydroxy-3 Butyl)-3 Dihydro-1,2 Oxy-2 Chinoxalin
- Hexyl-1 (Hydroxy-3 butyl)-3 Dihydro-1,2 Oxy-2 Chinoxalin
- (Pyridyl-3 Methyl)-1 (Hydroxy-3 Butyl)-3 Dihydro-1,2 Oxy-2 Chinoxalin
- (pyridyl-4 Methyl)-1 (Hydroxy-3 Butyl)-3 Dihydro-1,2 Oxy-2 Chinoxalin
- Crotyl-1 (Methylcarbamoyloxy-3 Propyl)-3 Dihydro-1,2 Oxy-2 Chinoxalin
- Propyl-1 (Isopropylcarbamoyloxy-3 Propyl)-3 Dihydro-1,2 Oxy-2 Chinoxalin
- Propyl-1 (Phenylcarbamoyloxy-3 Propyl)-3 Dihydro-1,2 Oxy-2 Chinoxalin
- (Acetoxy-2 Ethyl)-1 (Hydroxy-3 Propyl)-3 Dihydro-1,2 Oxy-2 Chinoxalin
- (Acetoxy-2 Ethyl)-1 (Hydroxy-3 butyl)-3 Dihydro-1,2 Oxy-2 Chinoxalin
- (Hydroxy-2 Ethyl)-1 (Hydroxy-3 Butyl)-3 Dihydro-1,2 Oxy-2 Chinoxalin
- (Hydroxy-2 ethyl)-1 (Hydroxy-3 Propyl)-3 Dihydro-1,2 Oxy-2 Chinoxalin
- Prenyl-1 (Hydroxy-5 Pentyl)-3 Dihydro-1,2 Oxy-2 Chinoxalin

3. Zubereitungsprozedur für chemische Verbindungen nach Patentanspruch 1 und 2, gekennzeichnet durch die Tatsache, daß die Verbindungen III durch Reaktion eines Orthophenylen-Diamin mit Acetosäure der Formel II gewonnen werden:
Formel II: 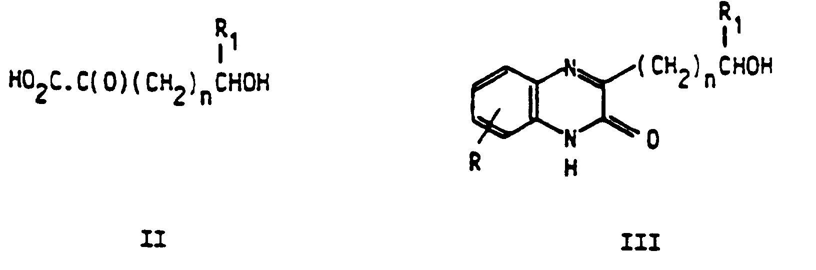 wobei n, R und R1 die gleiche Bedeutung wie in Patentanspruch 1 haben.

4. Zubereitungsprozedur für chemische Verbindungen nach Patentanspruch 1, 2 und 3, gekennzeichnet durch die Tatsache, daß die Reaktion der Verbindungen II mit Orthophylen-Diamin zur Herstellung von Verbindungen III in einer alkoholischen Lösung wie z.B. Äthanol, oder in einer sauren Lösung wie z.B. Ameisensäure gewonnen werden, wobei die Temperatur von Umgebungstemperatur bis zur Temperatur des Rückflusses des Lösungsmittels reichen kann.

5. Zubereitungsprozedur für chemische Verbindungen nach Patentanspruch 1 und 2, gekennzeichnet durch die Tatsache, daß die Verbindungen der allgemeinen Formel III in Verbindungen der allgemeinen Formel IV umgewandelt werden. 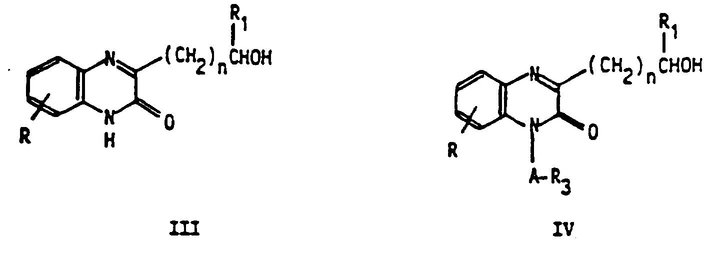 wobei n, R, R₁, A und R₃ die gleiche Bedeuting wie in Patentanspruch 1 haben.

6. Zubereitungsprozedur für chemische Verbindungen nach Patentanspruch 1, 2 und 3, gekennzeichnet durch die Tatsache, daß die Umwandlung von Verbindungen nach der allgemeinen Formel III in Verbindungen der allgemeinen Formel IV durch Reaktion der III-Verbindungen in Gegenwart von einer Base wie z.B. N atriumhydrid in einem aprotischen Lösungsmittel wie DMF mit einem Reagenz der Formel R₃-A-X gewonnen werden, worin X ein Atom von Jod, Brom, Chlor oder eine Gruppe wie Mesylat oder Tosylat ist.

7. Zubereitungsprozedur für chemische Verbindungen nach Patentanspruch 1 und 2, gekennzeichnet durch die Tatsache, daß man eine Verbindung der allgemeinen Formel IV mit eine Anhydrid der Formel (R₄CO)₂O reagieren läßt, und zwar vorzugsweise bei hoher Temperatur, um eine Verbindung der allgemeinen Formel I zu erhalten, worin R₂ für C(O)R₄ steht und n, R, R₁, A, R₃ und R₄ die gleiche Bedeutung wie in Patentanspruch 1 haben:

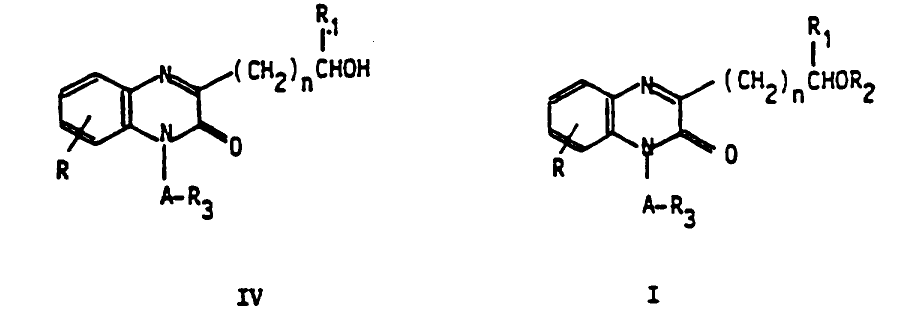

8. Zubereitungsprozedur für chemische Verbindungen nach Patentanspruch 1 und 2, gekennzeichnet durch:
- daß man eine Verbindung der allgemeinen Formel III mit einem Anhydrid der (R₄CO)₂O vorzugsweise bei hoher Temperatur reagieren läßt,
- daß man das derart erhaltene Reaktionsprodukt in Präsenz einer Base wie z.B. Natriumhydrid mit einem Reagenz der Formel R₃-A-X reagieren läßt, worin X ein Atom von Jod, Brom, Chlor oder eine Gruppe wie Mesylat oder Tosylat ist, um eine Verbindung der allgemeinen Formel I zu erhalten, in welcher R₂ für C(O)R₄ steht und n, R, R₁, A, R₃ sowie R₄ die gleiche Bedeutung haben wie in Patentanspruch 1:

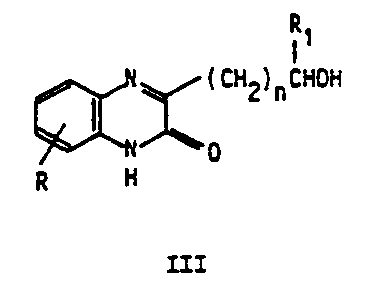

9. Zubereitungsprozedur für chemische Verbindungen nach Patentanspruch 1, 2 und 3, gekennzeichnet durch die Tatsache, daß man eine Verbindung der allgemeinen Formel IV mit Isocyanat der Formel R₅NCO in einem aprotischen Lösungsmittel wie Toluol reagieren läßt, um Verbindungen mit der allgemeinen Formel I zu erhalten, worin R₂ für -C(O)NHR₅ steht und n, R, R₁, A, R₃ sowie R₅ die gleiche Bedeutung haben wie in Patentanspruch 1.

10. Zubereitungsprozedur für chemische Verbindungen nach Patentanspruch 1, 2 und 3, gekennzeichnet durch die Tatsache, daß man eine Verbindung der allgemeinen Formel IV mit Phosgen, und dann mit einem Amin der Formel R₅NH₂ reagieren läßt, um Verbindungen mit der allgemeinen Formel I zu erhalten, worin R₂ für -C(O)R₅ steht und n, R, R₁, A, R₃ sowie R₅ die gleiche Bedeutung haben wie in Patentanspruch 1.

11. Zubereitungsprozedur für chemische Verbindungen nach Patentanspruch 1 und 2, gekennzeichnet durch die Tatsache, daß man eine Verbindung der allgemeinen Formel I, worin R₃ ein Hydroxylradikal ist, mit einem Überschuß der Formel (R₈CO)₂O reagieren läßt, und zwar vorzugsweise bei hohen Temperaturen, um eine Verbindung der allgemeinen Formel I zu erhalten, worin R₃ für eine -OC(O)R₈ -Gruppe steht und n, R, R₁, R₂, A sowie R₈ die gleiche Bedeutung haben wie in Patentanspruch 1.

12. Zubereitungsprozedur für chemische Verbindungen nach Patentanspruch 1 und 2, gekennzeichnet durch die Tatsache, daß man eine Verbindung der allgemeinen Formel I, worin R₃ ein Hydroxylradikal ist, mit einem Isocyanat der Formel R₉NCO in einem aprotischen Lösungsmittel wie Toluol oder Xylol reagieren läßt, um Verbindungen mit der allgemeinen Formel I zu erhalten, worin R₃ für -OC(O)NHR₉ steht und n, R, R₁, R₂, A sowie R₉ die gleiche Bedeutung haben wie in Patentanspruch 1.

13. Zubereitungsprozedur für chemische Verbindungen nach Patentanspruch 1 und 2, gekennzeichnet durch die Tatsache, daß die derart erhaltenen Verbindungen zur Herstellung von Arzneimitteln verwendet werden können.

## Claims (Claims for the following Contracting State(s): AT, BE, CH, LI, DE, DK, FR, GB, IT, LU, NL, SE)

1. New derivatives of dihydro-1,2 oxo-2 quinoxalines corresponding to the general formula I: 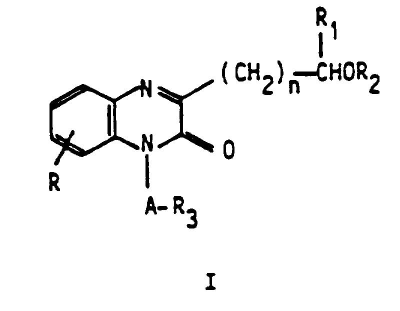 in which:
R represents an atom of hydrogen or halogen
R₁ represents an atom of hydrogen or a straight or branched alkyl radical in C₁-C₄
R₂ represents:
- an atom of hydrogen
- a C(O)R₄ radical
in which R₄ is a straight or branched alkyl group in C₁-C₄
- a C(O)NHR₅ radical
in which R₅ is a straight or branched alkyl group in C₁-C₄, or a phenyl group
A represents an alkylene chain in C₁-C₄
R₃ represents:
- an atom of hydrogen, an alkyl radical in C₁-C₄, a cycloalkyl radical in C₃-C₆, an alkynyl, nitryl, hydroxyl, carboxamid, pyridyl, phenyl group or phenyl replaced by an atom of halogen, an alkyl group in C₁-C₄, an alkoxy group in C₁-C₄, or a nitro group.
- an alkenvl radical with the formula: 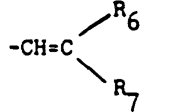 in which R₆, R₇ may be, independently of each other, an atom of hydrogen, an alkyl group in C₁-C₄, a phenyl group, or an alkoxy-carbonyl group
- a radical with the formula:
-OC(O)R₈
in which R₈ represents an alkyl radical in C₁-C₄
- a radical with the formula:
-OC(O)NHR₉
in which R₉ represents a straight or branched alkyl group in C₁-C₄, or a phenyl group
n may be valued from 2 to 4
as well as the organic salts or therapeutically acceptable minerals of these molecules.

2. Compounds of general formula I according to patent claim 1, characterized by the fact that the compounds are chosen from:
- prenyl-1 (hydroxy-3 propyl)-3 dihydro-1,2 oxo-2 quinoxaline
- allyl-1 (acetoxy-3 butyl)-3 dihydro-1,2 oxo-2 quinoxaline
- propyl 1 (methylcarbamoyloxy-3 propyl)-3 dihydro-1,2 oxo-2 quinoxaline
- (methylcarbamoyloxy-2 ethyl)-1 (hydroxy-3 butyl)-3 dihydro-1,2 Oxo-2 quinoxaline
- allyl-1 (hydroxy-3 propyl)-3 chloro-7 dihydro-1,2 oxo-2 quinoxaline
- propargyl-1 (hydroxy-3 propyl)-3 chloro-6(7) dihydro-1,2 oxo-2 quinoxaline
- allyl-1 (hydroxy-4 butyl)-3 dihydro-1,2 oxo-2 quinoxaline
- allyl-1 (hydroxy-3 propyl)-3 dihydro-1,2 oxo-2 quinoxaline
- allyl-1 (hydroxy-3 butyl)-3 dihydro-1,2 oxo-2 quinoxaline
- (methylcrotonyl)-1 (hydroxy-3 propyl)-3 dihydro-1,2 oxo-2 quinoxaline
- propyl-1 (hydroxy-3 propyl)-3 dihydro-1,2 oxo-2 quinoxaline
- crotyl-1 (hydroxy-3 propyl)-3 dihydro-1,2 oxo-2 quinoxaline
- propargyl-1 (hydroxy-3 propyl)-3 dihydro-1,2 oxo-2 quinoxaline
- cinnamyl-1 (hydroxy-3 propyl)-3 dihydro-1,2 oxo-2 quinoxaline
- cyclopropylmethyl-1 (hydroxy-3 propyl)-3 dihydro-1,2 oxo-2 quinoxaline
- cyanomethyl-1 (hydroxy-3 propyl)-3 dihydro-1,2 oxo-2 quinoxaline
- benzyl-1 (hydroxy-3 propyl)-3 dihydro-1,2 oxo-2 quinoxaline
- acetamido-1 (hydroxy-3 propyl)-3 dihydro-1,2 oxo-2 quinoxaline
- crotyl-1 (hydroxy-3 butyl)-3 dihydro-1,2 oxo-2 quinoxaline
- prenyl-1 (hydroxy-3 butyl)-3 dihydro-1,2 Oxo-2 quinoxaline
- cyclopropylmethyl-1 (hydroxy-3 butyl)-3 dihydro-1,2 oxo-2 quinoxaline
- (p.nitrobenzyl)-1 (hydroxy-3 propyl)-3 dihydro-1,2 oxo-2 quinoxaline
- propyl-1 (hydroxy-3 butyl)-3 dihydro-1,2 oxo-2 quinoxaline
- (p. methoxybenzyl)-1 (hydroxy-3 butyl)-3 dihydro-1,2 oxo-2 quinoxaline
- methyl-1 (hydroxy-3 butyl)-3 dihydro-1,2 oxo-2 quinoxaline
- butyl-1 (hydroxy-3 butyl)-3 dihydro-1,2 oxo-2 quinoxaline
- (pyridyl-2 methyl)-1 (hydroxy-3 butyl)-3 dihydro-1,2 oxo-2 quinoxaline
- pentyl-1 (hydroxy-3 butyl)-3 dihydro-1,2 oxo-2 quinoxaline
- hexyl-1 (hydroxy-3 butyl)-3 dihydro-1,2 oxo-2 quinoxaline
- (pyridyl-3 methyl)-1 (hydroxy-3 butyl)-3 dihydro-1,2 oxo-2 quinoxaline
- (pyridyl-4 methyl)-1 (hydroxy-3 butyl)-3 dihydro-1,2 oxo-2 quinoxaline
- crotyl-1 (methylcarbamoyloxy-3 propyl)-3 dihydro-1,2 oxo-2 quinoxaline
- propyl-1 (isopropylcarbamoyloxy-3 propyl)-3 dihydro-1,2 oxo-2 quinoxaline
- propyl-1 (phenylcarbamoyloxy-3 propyl)-3 dihydro-1 ,2 oxo-2 quinoxaline
- (acetoxy-2 ethyl)-1 (hydroxy-3 propyl)-3 dihydro-1,2 oxo-2 quinoxaline
- (acetoxy-2 ethyl)-1 (hydroxy-3 butyl)-3 dihydro-1,2 Oxo-2 quinoxaline
- (hydroxy-2 ethyl)-1 (hydroxy-3 butyl)-3 dihydro-1,2 oxo-2 quinoxaline
- (hydroxy-2 ethyl)-1 (hydroxy-3 propyl)-3 dihydro-1,2 oxo-2 quinoxaline
- prenyl-1 (hydroxy-5 pentyl)-3 dihydro-1,2 oxo-2 quinoxaline

3. Preparation procedure for chemicai compounds according to patent claims 1 and 2, characterized by the fact that the III compounds are obtained by the reaction of an orthophenylene diamine with an aceto-acid of formula II: 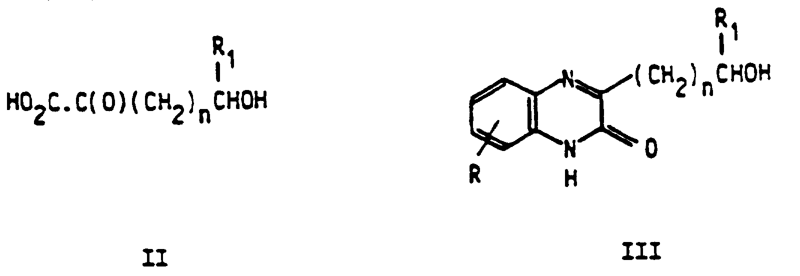 with n, R, R1 having the same meaning as in patent claim 1.

4. Preparation procedure for chemical compounds according to patent claims 1, 2 and 3, characterized by the fact that the reaction of the II compounds with the orthophenylene diamine to obtain the compounds of formula III is carried out in an alcohol solvent such as ethanol, or an acid solvent, such as acetic acid, at a temperature between ambient temperature and the temperature of the reflux of the solvent.

5. Preparation procedure for chemical compounds according to patent claims 1 and 2, characterized by the fact that the general formula III compounds are transformed into the compounds of general formula IV: 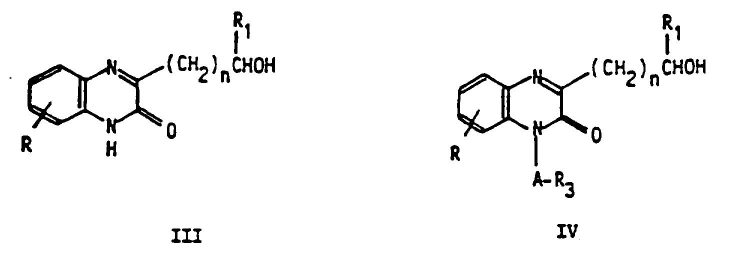 with n, R, R₁, A and R₃ having the same meaning as in patent claim 1.

6. Preparation procedure for chemical compounds according to patent claims 1, 2 and 3, characterized by the fact that the transformation of the general formula III compounds into the compounds of general formula IV is effected by the reaction of the III compounds in the presence of a base such as sodium hydride within an aprotic solvent such as DMF with a reagent with the formula R₃-A-X, in which X is an atom of iodine, bromine, chlorine or a group such as mesylate or tosylate.

7. Preparation procedure for chemical compounds according to patent claims 1 and 2, characterized by the fact that a compound of the general formula IV is made to react with an anhydride with the formula (R₄CO)₂O, preferably when hot, to obtain a compound with the general formula I, in which R₂ represents C(O)R₄ and n, R, R₁, A, R₃ and R₄ have the same meaning as in patent claim 1:

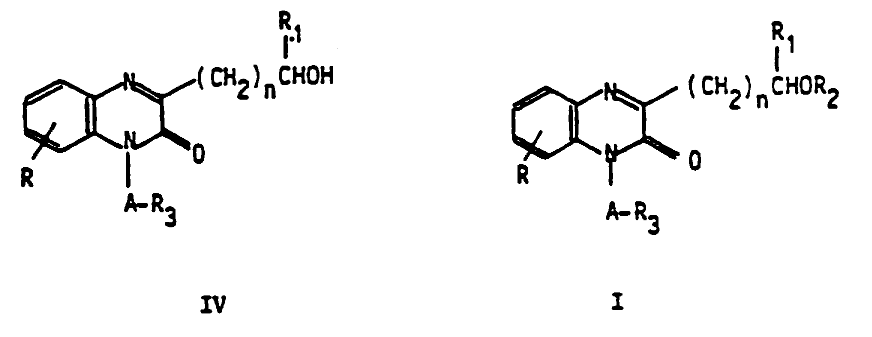

8. Preparation procedure for chemical compounds according to patent claims 1 and 2, characterized by the fact that:
- a compound of the general formula III is made to react with an anhydride with the formula (R₄CO)₂O, preferably when hot,
- the product obtained in this way is made to react, in the presence of a base such as sodium hydride, to a reactive with the formula R₃-A-X, in which X is an atom of iodine, bromine, chloride or a group such as mesylate or tolylate, to obtain a compound of the general formula I, in which R₂ represents C(O)R₄ and n, R, R₁, A, R₃ and R₄ have the same meaning as in patent claim 1:

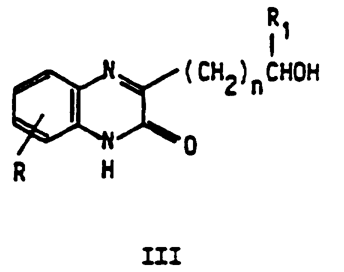

9. Preparation procedure for chemical compounds according to patent claims 1, 2 and 3, characterized by the fact that a compound of the general formula IV is made to react with an isocyanate with the formula R₅NCO in an aprotic solvent such as toluene to obtain compounds with the general formula I, in which R₂ represents -C(O)NHR , and n, R, R₁, A, R₃ and R₅ have the same meaning as in patent claim 1:

10. Preparation procedure for chemical compounds according to patent claims 1, 2 and 3, characterized by the fact that a compound of the general formula IV is made to react with phosgene gas and then with an amine with the formula R₅NH₂ to obtain compounds with the general formula I, in which R₂ represents -C(O)NHR₅, and n, R, R₁, A, R₃ and R₅ have the same meaning as in patent claim 1:

11. Preparation procedure for chemical compounds according to patent claims 1 and 2, characterized by the fact that a compound of the general formula I, in which R₃ represents a hydroxyl radical, is made to react with a surplus of anhydride of the formula (R₈CO)₂O, preferably when hot, to obtain a compound with the general formula I, in which R₃ represents a -OC(O)R₈ group and n, R, R₁, R₂, A, and R₈ have the same meaning as in patent claim 1.

12. Preparation procedure for chemical compounds according to patent claims 1 and 2, characterized by the fact that a compound of the general formula I, in which R₃ represents a hydroxyl radical, is made to react with an isocyanate with the formula R₉NCO in an aprotic solvent such as toluene or xylene to obtain a compound with the general formula I, in which R₃ represents an -OC(O)NHR₉, and n; R, R₁, R₂ , A and R₉ have the same meaning as in patent claim 1:

13. Medicines for the treatment of chronic obstructive bronchopneumopathies, respiratory deficiency, emphysema, and cardiovascular pathologies relating to cardiac deficiency, containing the compounds defined according to one of the patent claims 1 and 2.

14. Pharmaceutical composition characterized by the fact that it contains a compound of one of the patent claims 1 and 2.

15. Pharmaceutical composition characterized by the fact that it contains a compound of one of the patent claims 1 and 2 in association with any appropriate excipient.

16. Pharmaceutical composition characterized by the fact that it contains a compound of one of the patent claims 1 and 2 in association with another active principle.

## Claims (Claims for the following Contracting State(s): ES, GR)

1. Preparation procedure for derivatives of dihydro-1,2 oxo-2 quinolaxines corresponding to the general formula I: 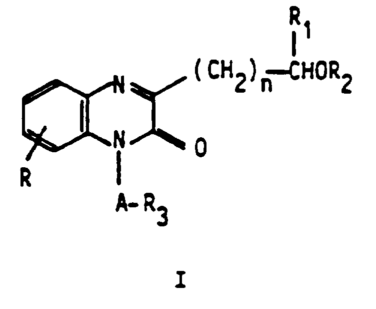 in which:
R represents an atom of hydrogen or halogen
R₁ represents an atom of hydrogen or a straight or branched alkyl radical in C₁-C₄
R₂ represents:
- an atom of hydrogen
- a C(O)R₄ radical
in which R₄ is a straight or branched alkyl group in C₁-C₄
- a C(O)NHR₅ radical
in which R₅ is a straight or branched alkyl group in C₁-C₄, or a phenyl group
A represents an alkylene chain in C₁-C₄
R₃ represents:
- an atom of hydrogen, an alkyl radical in C₁-C₄, a cycloalkyl radical in C₃-C₆, an alkynyl, nitryl, hydroxyl, carboxamid, pyridyl, phenyl group or phenyl replaced by an atom of halogen, an alkyl group in C₁-C₄, an alkoxy group in C₁-C₄, or a nitro group.
- an alkenyl radical with the formula: 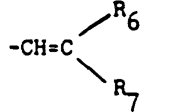 in which R₆, R₇ may be, independently of each other, an atom of hydrogen, an alkyl group in C₁-C₄, a phenyl group, or an alkoxy-carbonyl group
- a radical with the formula:
-OC(O)R₈
in which R₈ represents an alkyl radical in C₁-C₄
- a radical with the formula:
-OC(O)NHR₉
in which R₉ represents a straight or branched alkyl group in C₁-C₄, or a phenyl group
n may be valued from 2 to 4
as well as the organic salts or therapeutically acceptable minerals of these molecules.

2. Preparation procedure for the compound of general formula I according to patent claim 1 characterized by the fact that the compounds are chosen from:
- prenyl-1 (hydroxy-3 propyl)-3 dihydro-1,2 oxo-2 quinoxaline
- allyl-1 (acetoxy-3 butyl)-3 dihydro-1,2 oxo-2 quinoxaline
- propyl-1 (methylcarbamoyloxy-3 propyl)-3 dihydro-1,2 oxo-2 quinoxaline
- (methylcarbamoyloxy-2 ethyl)-1 (hydroxy-3 butyl)-3 dihydro-1,2 oxo-2 quinoxaline
- allyl-1 (hydroxy-3 propyl)-3 chloro-7 dihydro-1,2 oxo-2 quinoxaline
- propargyl-1 (hydroxy-3 propyl)-3 chloro-6(7) dihydro-1,2 oxo-2 quinoxaline
- allyl-1 (hydroxy-4 butyl)-3 dihydro-1,2 oxo-2 quinoxaline
- allyl-1 (hydroxy-3 propyl)-3 dihydro-1,2 oxo-2 quinoxaline
- allyl-1 (hydroxy-3 butyl)-3 dihydro-1,2 oxo-2 quinoxaline
- (methylcrotonyl)-1 (hydroxy-3 propyl)-3 dihydro-1,2 oxo-2 quinoxaline
- propyl-1 (hydroxy-3 propyl)-3 dihydro-1,2 oxo-2 quinoxaline
- crotyl-1 (hydroxy-3 propyl)-3 dihydro-1,2 oxo-2 quinoxaline
- propargyl-1 (hydroxy-3 propyl)-3 dihydro-1,2 oxo-2 quinoxaline
- cinnamyl-1 (hydroxy-3 propyl)-3 dihydro-1,2 oxo-2 quinoxaline
- cyclopropylmethyl-1 (hydroxy-3 propyl)-3 dihydro-1,2 oxo-2 quinoxaline
- cyanomethyl-1 (hydroxy-3 propyl)-3 dihydro-1,2 oxo-2 quinoxaline
- benzyl-1 (hydroxy-3 propyl)-3 dihydro-1,2 oxo-2 quinoxaline
- acetamido-1 (hydroxy-3 propyl)-3 dihydro-1,2 oxo-2 quinoxaline
- crotyl-1 (hydroxy-3 butyl)-3 dihydro-1,2 oxo-2 quinoxaline
- prenyl-1 (hydroxy-3 butyl)-3 dihydro-1,2 oxo-2 quinoxaline
- cyclopropylmethyl-1 (hydroxy-3 butyl)-3 dihydro-1,2 oxo-2 quinoxaline
- (p.nitrobenzyl)-1 (hydroxy-3 propyl)-3 dihydro-1,2 oxo-2 quinoxaline
- propyl-1 (hydroxy-3 butyl)-3 dihydro-1,2 oxo-2 quinoxaline
- (p. methoxybenzyl)-1 (hydroxy-3 butyl)-3 dihydro-1,2 oxo-2 quinoxaline
- methyl-1 (hydroxy-3 butyl)-3 dihydro-1,2 oxo-2 quinoxaline
- butyl-1 (hydroxy-3 butyl)-3 dihydro-1,2 oxo-2 quinoxaline
- (pyridyl-2 methyl)-1 (hydroxy-3 butyl)-3 dihydro-1,2 oxo-2 quinoxaline
- pentyl-1 (hydroxy-3 butyl)-3 dihydro-1,2 oxo-2 quinoxaline
- hexyl-1 (hydroxy-3 butyl)-3 dihydro-1,2 oxo-2 quinoxaline
- (pyridyl-3 methyl)-1 (hydroxy-3 butyl)-3 dihydro-1,2 oxo-2 quinoxaline
- (pyridyl-4 methyl)-1 (hydroxy-3 butyl)-3 dihydro-1,2 oxo-2 quinoxaline
- crotyl-1 (methylcarbamoyloxy-3 propyl)-3 dihydro-1,2 oxo-2 quinoxaline
- propyl-1 (isopropylcarbamoyloxy-3 propyl)-3 dihydro-1,2 oxo-2 quinoxaline
- propyl-1 (phenylcarbamoyloxy-3 propyl)-3 dihydro-1,2 oxo-2 quinoxaline
- (acetoxy-2 ethyl)-1 (hydroxy-3 propyl)-3 dihydro-1,2 oxo-2 quinoxaline
- (acetoxy-2 ethyl)-1 (hydroxy-3 butyl)-3 dihydro-1,2 oxo-2 quinoxaline
- (hydroxy-2 ethyl)-1 (hydroxy-3 butyl)-3 dihydro-1,2 oxo-2 quinoxaline
- (hydroxy-2 ethyl)-1 (hydroxy-3 propyl)-3 dihydro-1,2 oxo-2 quinoxaline
- prenyl-1 (hydroxy-5 pentyl)-3 dihydro-1,2 oxo-2 quinoxaline

3. Preparation procedure for chemical compounds according to patent claims 1 and 2, characterized by the fact that the III compounds are obtained by the reaction of an orthophenylene diamine with an aceto-acid of formula II: 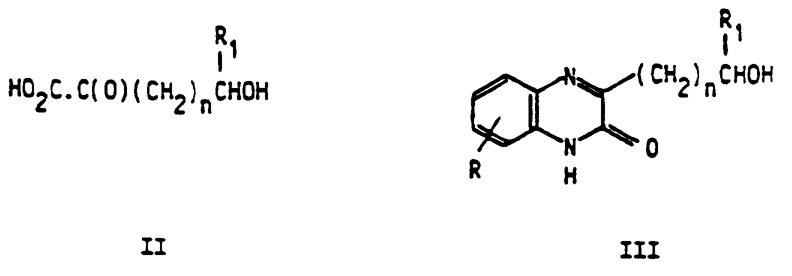 with n, R, R1 having the same meaning as in patent claim 1.

4. Preparation procedure for chemical compounds according to patent claims 1, 2 and 3, characterized by the fact that the reaction of the II compounds with the orthophenylene diamine to obtain the compounds of formula III is carried out in an alcohol solvent such as ethanol, or an acid solvent, such as acetic acid, at a temperature between ambient temperature and the temperature of the reflux of the solvent.

5. Preparation procedure for chemical compounds according to patent claims 1 and 2, characterized by the fact that the general formula III compounds are transformed into the compounds of general formula IV: 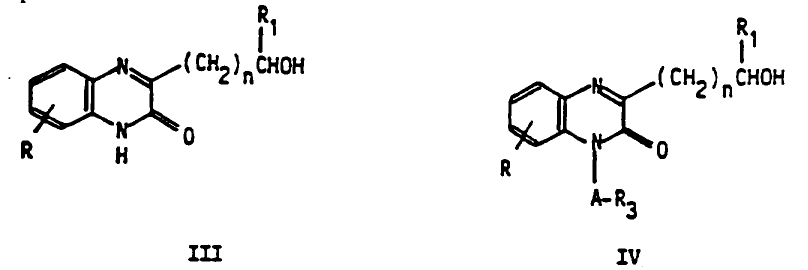 with n, R, R₁, A and R₃ having the same meaning as in patent claim 1.

6. Preparation procedure for chemical compounds according to patent claims 1, 2 and 3, characterized by the fact that the transformation of the general formula III compounds into the compounds of general formula IV is effected by the reaction of the III compounds in the presence of a base such as sodium hydride within an aprotic solvent such as DMF with a reagent with the formula R₃-A-X, in which X is an atom of iodine, bromine, chlorine or a group such as mesylate or tosylate.

7. Preparation procedure for chemical compounds according to patent claims 1 and 2, characterized by the fact that a compound of the general formula IV is made to react with an anhydride with the formula (R₄CO)₂O, preferably when hot, to obtain a compound with the general formula I, in which R₂ represents C(O)R₄ and n, R, R₁, A, R₃ and R₄ have the same meaning as in patent claim 1:

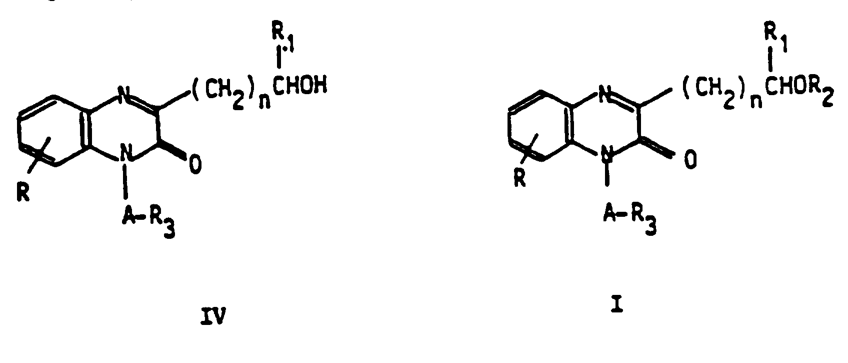

8. Preparation procedure for chemical compounds according to patent claims 1 and 2, characterized by the fact that:
- a compound of the general formula III is made to react with an anhydride with the formula (R₄CO)₂O, preferably when hot,
- the product obtained in this way is made to react, in the presence of a base such as sodium hydride, to a reactive with the formula R₃-A-X, in which X is an atom of iodine, bromine, chloride or a group such as mesylate or tolylate, to obtain a compound of the general formula I, in which R₂ represents C(O)R₄ and n, R, R₁, A, R₃ and R₄ have the same meaning as in patent claim 1:

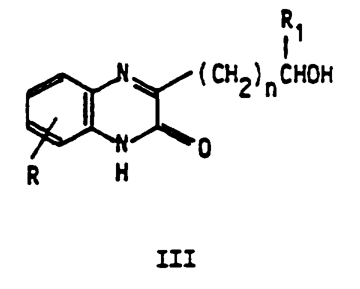

9. Preparation procedure for chemical compounds according to patent claims 1, 2 and 3, characterized by the fact that a compound of the general formula IV is made to react with an isocyanate with the formula R₅NCO in an aprotic solvent such as toluene to obtain compounds with the general formula I, in which R₂ represents -C(O)NHR , and n, R, R₁, A, R₃ and R₅ have the same meaning as in patent claim 1:

10. Preparation procedure for chemical compounds according to patent claims 1, 2 and 3, characterized by the fact that a compound of the general formula IV is made to react with phosgene gas and then with an amine with the formula R₅NH₂ to obtain compounds with the general formula I, in which R₂ represents -C(O)NHR₅, and n, R, R₁, A, R₃ and R₅ have the same meaning as in patent claim 1:

11. Preparation procedure for chemical compounds according to patent claims 1 and 2, characterized by the fact that a compound of the general formula I, in which R₃ represents a hydroxyl radical, is made to react with a surplus of anhydride of the formula (R₈CO)₂O, preferably when hot, to obtain a compound with the general fonnula I, in which R₃ represents a -OC(O)R₈ group and n, R, R₁, R₂, A, and R₈ have the same meaning as in patent claim 1.

12. Preparation procedure for chemical compounds according to patent claims 1 and 2, characterized by the fact that a compound of the general formula I, in which R₃ represents a hydroxyl radical, is made to react with an isocyanate with the formula R₉NCO in an aprotic solvent such as toluene or xylene to obtain a compound with the general formula I, in which R₃ represents an -OC(O)NHR₉, and n, R, R₁, R₂ , A and R₉ have the same meaning as in patent claim 1:

13. Preparation procedure for chemical compounds according to patent claims 1 and 2, characterized by the fact that the compounds obtained are used in the preparation of medicines.
